# EUROPEAN PATENT SPECIFICATION

(11) **EP 0 041 491 B1**
(45) Date of publication and mention of the grant of the patent: **30.01.1985**
(21) Application number: 81850095.1
(22) Date of filing: 27.05.1981
(51) Int. Cl.: C07C 93/06, A61K 31/22

(54) **New para-substituted 3-phenoxy-1-alkylamino-propanol-2-s having beta receptor blocking properties**
Parasubstituierte 3-Phenoxy-1-alkylaminopropan-2-ole mit beta-Rezeptor blockierenden Eigenschaften
3-Phénoxy-1-alkylaminopropan-2-ols substitués en position para et ayant la capacité de bloquer les bêta-récepteurs

(30) Priority: 02.06.1980 SE 8004087
(43) Date of publication of application: 09.12.1981
(73) Proprietor: Aktiebolaget Hässle, S-431 83 Mölndal (SE)
(72) Inventor: Carlsson, Enar Ingemar, S-421 68 Västra Frölunda (SE); Gustafsson, Bill Benjamin Rudolf, S-517 00 Bollebygd (SE); Lundgren, Bo Torsten, S-430 30 Frillesäs (SE)
(74) Representative: Wurm, Bengt Runio

## Description

### Technical field

The present invention relates to new compounds having valuable properties in treating acute myocardial infarction and patients undergoing different kinds of surgery while exerting beta receptore blocking activity, process for their preparation, and pharmaceutical preparations containing said compounds.

The object of the present invention is to obtain new compounds with beta adrenoceptor blocking activity and with such a short biological halflife that the degree of beta adrenoceptor blockade easily can be controlled by means of the parenteral administration rate. Such compounds can then be used in the vulnerable phase of actue myocardial infarction in order to reduce infarct size and also to prevent arrhythmias. The compounds may also be used as antiarrhythmics during various surgical procedures.

Generally, the new compounds can be used in the treatment of all indications where A-receptor blockers are used with the restriction that they can only be used by way of intravenous and topic administrations.

### Background of the invention

Compounds of the general structure 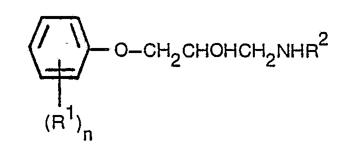 where n is an integer 1 to 3, and R' is i.a. any substituent of the group alkyl, alkenyl, alkinyl, alkoxy, alkenyloxy, alkinyloxy, halogen, nitro, cyano, alkoxyalky, alkoxyalkoxy, aminoalkylamino, optionally substituted with one or two alkyl groups, aminocarbonylaminoalkyl, optionally substituted with one or two alkyl groups, aryl, aralkyl, aralkoxy and aroyl, and R² is straight or branched alkyl, or hydroxyalkyl, cycloalkyl, or phenylalkyl, or phenoxyalkyl optionally substituted with alkyl, alkoxy, hydroxy, cyano, cyanomethyl, hydroxymethyl, or trifluoromethyl, are known to possess beta-adrenoceptor blocking activity whereby they are used in treating angina pectoris, heart arrhythmias, hypertension, and glaucoma.

DE-A-2 736 106 (Hexachimie S.A.) discloses compounds under the formula 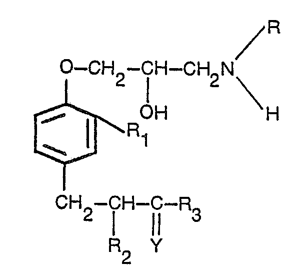 wherein =C=Y is =CH₂, =C=O or =C(H)OH and R₃ is straight or branched alkyl or cyclopropyl, said compounds having a β₁-receptor blocking activity. Duration data given in table 29 indicate that the compounds are not shortacting.

US-C-3 998 790 (Aktiebolaget Hâssle) discloses compounds of the formula 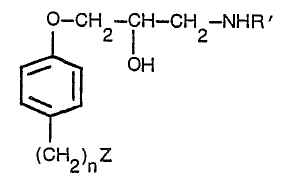 wherein R' is isopropyl or tert. butyl, Z is -OR", -SR" or -NHCOOR", R" being alkyl and n is 1, 2 or 3. The compounds are selective β-receptor blockers having a normal duration of action. The compound wherein R' is isopropyl, Z is -OR", R" is methyl and n is 2 is named metoprolol (Seloken®, Beloc@) and is used in oral administration once or twice a day.

GB-C-1 358 803 (Lipha) discloses compounds under the formula 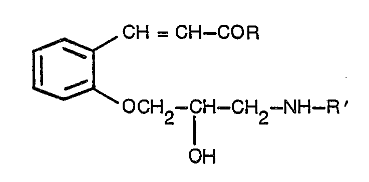 wherein R is hydroxy or alkoxy i.a. said compounds being β-receptor blockers administerable orally or parenterally.

GB-C-1 285 038 (ICI) discloses compounds of the formula 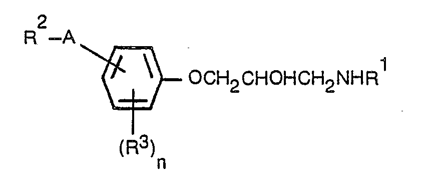 wherein R¹ is alkyl or hydroxyalkyl, R² is acyl or a functional derivative of the carboxy radical such as cyano or alkoxycarbonyl i.a., A is alkylene or alkenylene, n is 1 or 2 and R³ is hydrogen or a substituent. Among thereapeutically useful compounds specifically disclosed is 3-isopropylamino-1-(2-methoxy-4-β-methoxycarbonyl vinylphenoxy)-2-propanol (Example 11) and 1-(4-β-ethoxycarbonylvinyl-2-methoxyphenoxy)-3-isopropylaminopropan-2-ol (Example 17). However, the invention focuses on compounds wherein R² is carbamoyl or substituted carbamoyl. Further disclosed are 3-isopropylamino-1-p-methoxycarbonylmethyiphenoxy-2-propanol and 1₋p-ethoxycarbonylmethyl- phenoxy-3-isopropylamino-2-propanol which are used as intermediates in Examples 9 and 26, respectively. The disclosed therapeutic utility is as β-adrenergic blocking agents useful against heart diseases. There is no suggestion for obtaining compounds with a short duration of action.

Further, there are several indications that treatment with beta-adrenoceptor blockers in the early phase of acute myocardial infarction may reduce infarct size (c.f. Waagstein et al., 1977 In: Acute and Long Term Medical Management of Myocardial Ischaemia. Eds: A Hjalmarsson and L Wilhelmsen, Lindgren Soner AB, Mölndal, pp 346-357) and possibly also prevent arrhythmias.

However, since the first attempt to use beta-adrenoceptor blockers in acute myocardial infarction about 15 years ago, the treatment has been viewed with some doubt due to alarming reports of side effects (cf Snow, P. J.: Effects of propranolol in myocardial infarction. Lancet 2: 551-553, 1965), and it is evident that treatment with beta-adrenoceptor blockers may be harmful to some patients with low cardiac output, severe heart failure and conduction disturbances. At present therefore very strict criteria are followed for the selection of patients for treatment with beta-adrenoceptor blockers during the acute phase of myocardial infarction. And even then some patients with myocardial infarction do not tolerate the beta-adrenoceptor blocker.

A great advantage in this connection would be to use a very shortacting beta-adrenoceptor blocker which could be given in a continuous intravenous infusion.

The degree of beta-blockade could then be easily controlled by changes of the infusion rate. Furthermore, if beta-blockade is not tolerated by the patient the infusion could be stopped and the effect will then disappear within a few minutes due to the short half-life of the drug.

There would also be a great advantage to have a shortacting beta-adrenoceptor blocker available in anaesthesiology and in the intensive care units. For example, arrhythmias are common in connection with intubation and laryngoscopy. It is known that these arrhythmias can be treated with beta-adrenoceptor blockers. The now available beta-blockers, however, are long acting and the blockade remains longer than needed.

The present para-substituted-3-phenoxy-1-alkylamino-propanol-2-s of this patent application are all very shortacting, potent, beta₁-selective blockers which can be used in the indications mentioned above. These compounds should be infused intravenously at an infusion rate of 0.5-1.5 µmol/kg bodyweight x minute.

### Disclosure of the invention

It has been found that the compounds of the general formula I 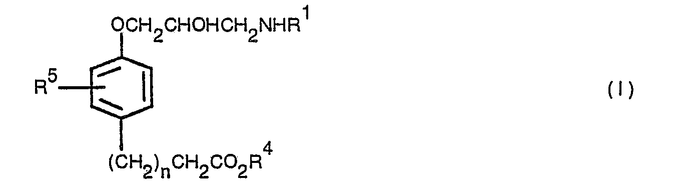 wherein R^{I} is selected from the group consisting of isopropyl and tert. butyl, R⁵ is selected from the group consisting of hydrogen, halogen, lower alkyl having up to 4 carbon atoms, lower alkenyl having up to 4 carbon atoms, and lower alkoxy having up to 4 carbon atoms, R⁴ is selected from the group consisting of alkyl having up to 7 carbon atoms, alkoxyalkyl having up to 8 carbon atoms, cycloalkyl having 3-6 carbon atoms, phenyl, arylalkyl having up to 10 carbon atoms, and hydroxyalkyl having up to 7 carbon atoms, and n is an integer 1 or 2, and pharmaceutically acceptable acid addition salts thereof, and are shortacting, potent beta₁-selective adrenoceptor blockers.

Alkyl R⁴ is an alkyl having up to 7 carbon atoms, preferably up to 5 carbon atoms, which alkyl group can be straight or branched. Thus alkyl R⁴ can be methyl, ethyl, n-propyl, isopropyl, n-butyl, isobutyl, or n-pentyl.

Alkoxyalkyl R⁴ is an alkoxyalkyl having up to 4 carbon atoms in each alkyl part, which alkyl parts can be straight or branched. Alkoxyalkyl R⁴ can thus be methoxymethyl, methoxyethyl, methoxypropyl, ethoxymethyl, ethoxyethyl, or isopropoxyethyl.

Cycloalkyl R⁴ is a cycloalkyl having 3 to 6 carbon atoms. Cycloalkyl R⁴ can thus be cyclopropyl, cyclobutyl, cyclopentyl, or cyclohexyl, but can also include cyclopropylmethyl, and 2-cyclopropylethyl.

Arylalkyl R⁴ is any arylalkyl having up to 10 carbon atoms. Arylalkyl R⁴ can thus be benzyl, 2-phenylethyl, 3-phenylpropyl, or 2-(o, m, or p-tolyl)ethyl.

Hydroxyalkyl R⁴ is a hyudroxyalkyl having up to 7 carbon atoms in the alkyl part. Hydroxyalkyl R⁴ can thus be 2-hydroxyethyl, 2-hydroxy-1-methylethyl, 3-hydroxypropyl, 4-hydroxybutyl, 3-hydroxy-1-methylpropyl, or 1,1-dimethyl-2-hydroxyethyl.

Halogen R⁵ is chlorine, fluorine, bromine, or iodine.

Alkyl R⁵ is a lower alkyl having up to 4 carbon atoms. Alkyl R⁵ is, e.g. methyl, ethyl, isopropyl, or tert.-butyl.

Alkenyl R⁵ is a lower alkenyl having up to 4 carbon atoms. Alkenyl R⁵ is, e.g. allyl.

Alkoxy R⁵ is a lower alkoxy having up to 4 carbon atoms. Alkoxy R⁵ is, e.g. methoxy, ethoxy or iso- propoxy.

R⁵ is preferably present in ortho-position to the alkylaminopropanol-2 side chain, but can under certain circumstances also be present in meta position to said side chain.

Specific compounds of the invention are:
1. Methyl 3-[4-(2-hydroxy-3-isopropylaminopropoxy)phenyl]propanoate;
2. Ethyl 3-[4-(2-hydroxy-3-isopropylaminopropoxy)phenyl]propanoate;
3. Methyl 3-14-(2-hydroxy-3-isopropylaminopropoxy)-3-methoxyphenyl]propanoate;
4. Propyl 3-[4-(2-hydroxy-3-isopropylaminopropoxy)phenyl]propanoate;
5. 2-Methoxyethyl 3-[4-(2-hydroxy-3-isopropylaminopropoxy)phenyl]propanoate;
6. Ethyl 4-[4-(2-hydroxy-3-isopropylaminopropoxy)phenyl]butanoate;
7. 2-Methoxyethyl 4-[4-(2-hydroxy-3-isopropylaminopropoxy)phenyl]butanoate;
8. Isopropyl 3-[4-(2-hydroxy-3-isopropylaminopropoxy)phenyl]propanoate;
9. Ethyl 4-[3-bromo-4-(2-hydroxy-3-isopropylaminopropoxy)phenyl]butanoate;
10. Ethyl-3-[3-chloro-4-(2-hydroxy-3-isopropylaminopropoxy)phenyl]propanoate;
11. Ethyl 3-[3-fluoro-4-(2-hydroxy-3-tert.-butylaminopropoxy)phenyl]propanoate;
12. 2-Phenylethyl 3-[4-(2-hydroxy-3-isopropylaminopropoxy)phenyl]propanoate;
13. Phenyl 3-[4-(2-hydroxy-3-isopropylaminopropoxy)phenyl]prop.anoate;
14. Ethyl 3-[3-bromo-4-(2-hydroxy-3-isopropylaminopropoxy)phenyl]propanoate;
15. Ethyl 3-(4-(2-hydroxy-3-isopropylaminopropoxy)-3-methoxyphenylJpropanoate;
16. Ethyl 3-[3-ethoxy-4-(2-hydroxy-3-isopropylaminopropoxy)phenyl]propanoate;
17. Isopropyl 3-[4-(2-hydroxy-3-isopropylaminopropoxy]-3-methoxyphenyl]propanoate.
18. Ethyl 3-[3-fluoro-4-(2-hydroxy-3-isopropylaminopropoxy)phenyl]propanoate.

These new compounds can be used at the treatment of acute myocardial infarctions and arrhythmias. One may also use them as intermediates at the preparation of other valuable pharmaceutical compounds.

Salt forming acids may be used in preparing therapeutically acceptable salts of the compounds. These are: hydrohalogen acids, sulfuric acide, phosphoric acid, nitric acid, perchloric acid, aliphatic, alicyclic, aromatic or heterocyclic carboxy or sulfonic acids, such as formic, acetic, propionic, succinic, glycolic, lactic, malic, tartaric, citric, ascorbic, maleic, hydroxymaleic, or pyrovic acid, phenylacetic, benzoic, p-aminobenzoic, anthranilic, p-hydroxybenzoic, salicyclic or p-aminosalicyclic acid, embonic acid, methanesulfonic, ethanesulfonic, hydroxyethanesulfonic, ethylenesulfonic, halogenbenzenesulfonic, toluenesulfonic, naphthylsulfonic, or sulfanilic acid, methionine, tryptophane, lysine or arginine.

The substances are intended to be administered parenterally for acute and chronic treatment of above mentioned cardio-vascular disorders.

The biological effects of the new compounds have been tested, and the different tests carried out will be shown and explained below.

The new compounds are obtained according to methods known per se. Thus, a compound of formula II 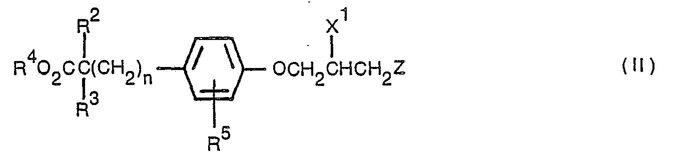 wherein R² and R³ are both hydrogen, and R⁴, R⁵ and n have the meanings given above, X^{I} is a hydroxy group, Z is hydrogen or a reactive, esterified hydroxy group, or X¹ and Z together form an epoxy group, is reacted with an amine of the formula III 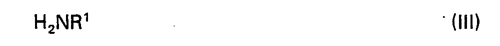 wherein R' has the meaning given above.

A reactive, esterified hydroxy group is particularly a hydroxy group esterified with a strong, inorganic or organic acid, preferably a hydrohalogen acid, as hydrochloric acid, hydrobromic acid, or hydroiodic acid, further sulfuric acid or a strong organic sulfonic acid, e.g. benzenesulfonic acid, 4-bromobenzenesulfonic acid, or 4-toluenesulfonic acid. Thus, Z is preferably chloro, bromo or iodo.

This reaction is carried out in a common way. At the use of a reactive ester as a starting material the preparation takes place preferably in the presence of a basic condensating agent and/or with an excess of an amine. Suitable basic condensating agents are e.g. alkalimetal hydroxides as sodium or potassium hydroxide, alkalimetal carbonates as potassium carbonate and alkalimetal alcoholates as sodium methylate, potassium ethylate and potassium tert.-butylate.

When Z is -OH the reaction is carried out in the presence of a metal catalyst, as Raney nickel.

The reaction is preferably carried out in an alkanol having 1 to 4 carbon atoms by refluxing the reactants in said solvent for a time long enough to give the compound of formula I, generally 1 to 12 hours. However, the reaction can be carried out in the presence of an excess of amine alone. The reaction can also take place in an autoclave.

Further, a compound of formula IV 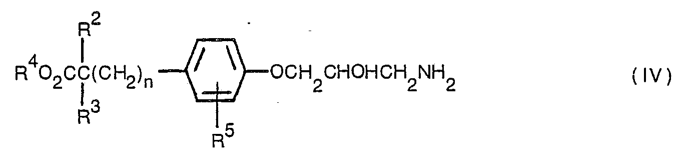 wherein R², R³, R⁴, R⁵ and n have the meanings given above, is reacted with a compound of the formula V 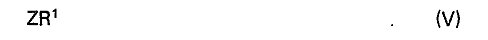 wherein Z and R^{I} have the same meanings as given above.

This reaction is carried out in a common way, preferably in the presence of a basic condensating agent and/or an excess of an amine. Suitable basic condensating agents are e.g. alkaline alcoholates, preferably sodium or potassium alcoholate, or also alkaline carbonates as sodium or potassium carbonate.

This reaction is carried out with or without the presence of an alkanol having 1 to 3 carbon atoms in an autoclave being heated to 100 to 130°C for 5 to 15 hours.

Compound V above can be replaced with CH₃-CH=CH₂ or (CH₃)₂C=CH₂, whereby the alkylation reaction is carried out via aminomercuration.

Further, a compound of formula VI 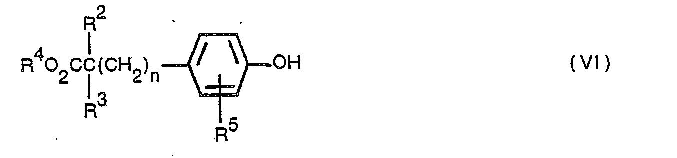 wherein R², R³, R⁴, R⁵ and n have the same meanings as given above is reacted with a compound of formula VII 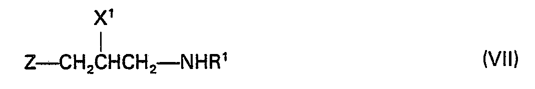 wherein R¹, Z and X¹ have the same meanings as given above, except for Z being OH.

This reaction is carried out in a common way. In those cases where reactive esters are used as starting material, the compound of formula VI may suitably be used in the form of its metalphenolate as alkalimetalphenolate, preferably sodiumphenolate, or one works in the presence of an acid binding agent, preferably a condensating agent, which can form a salt of the compound of formula VI as an alkalimetal alcoholate.

This reaction is preferably carried out in an alkanol having 1 to 3 carbon atoms in an autoclave being heated to 80 to 100°C for 1 to 15 hours.

Further, a compound of formula VI 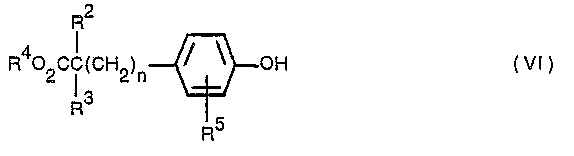 wherein R², R³, R⁴, R⁵ and n have the same meanings as given above, is reacted with a compound of formula VIII 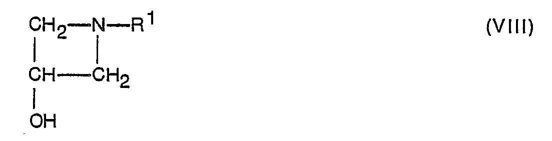 wherein R^{I} has the meaning given above.

This reaction is carried out in a common way. Thus, the reaction is carried out under alkaline conditions in a suitable solvent, as benzylalcohol by boiling the reaction mixture for some hours. Thereby the phenol is primarily converted to its metalphenolate as alkalimetalphenolate before it is added to the acetidinol of formula VIII.

Further, one may split off a residue from a compound of formula I above, in which the nitrogen atom of the amino group and/or the hydroxy groups have attached thereto a splitable residue.

Such splitable residues are especially those which are splitable by solvolysis, reduction, pyrolysis or fermentation.

At this splitting off of a residue the para-substituent 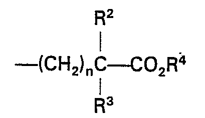 can be formed from another substituent X² containing one or more at these conditions so reacting functional groups. Such functional groups are e.g. benzylic carbonyl, hydroxyl or halogen, or carbon-carbon unsaturation.

Residues splitable by means of hydrolysis are e.g. an acyl residue, which, when present, also can be functionally varied carboxy groups, e.g. oxycarbonyl residues, as alkoxycarbonyl residues, e.g. tert. butoxycarbonyl residue, or ethoxycarbonyl residue, aralkoxycarbonyl residues as phenylloweralkoxy- carbonyl residues, e.g. a carbobenzyloxy residue, halogencarbonyl residue, e.g. a chlorocarbon residue, and carbamoyl groups. Further, arylsulphonyl residues as toluenesulfonyl or bromobenzenesulfonyl residues and possibly as halogenated, as fluorinated lower alkanoyl residues as formyl-, acetyl- or trifluoroacetyl residues or a benzyl residue or cyano groups or silyl residues, as trimethylsilyl residue.

Of the above mentioned residues present at the hydroxy groups, which residues are splitable by hydrolysis, preferably the oxycarbonyl residues and the loweralkanoyl residues or the benzoyl residues are used.

Besides the above mentioned also double-bound residues, which are splitable at the amino group by hydrolysis are used, e.g. alkylidene or benzylidene residue or a phosphorylidene group as a triphenyl- phosphorylidene group, whereby the nitrogen atom then obtains a positive charge.

Residues splitable at the amino group by hydrolysis are furthermore divalent residues as in occurring cases substituted methylene. As substituents on the methylene residues any organic residue may be used, whereby it does not matter at the hydrolysis which compound is the substituent to the methylene residue. As methylene substituents e.g. aliphatic or aromatic residues as alkyl as mentioned above, aryl e.g. phenyl or pyridyl may be used. The hydrolysis may be carried out in any common way, suitably in a basic or preferably in an acid medium.

Compounds having residues being splitable by hydrolysis are also the compounds according to formula IX 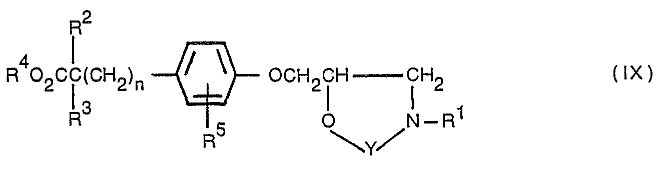 wherein R', R², R³, R⁴, R⁵ and n have the same meanings as given above and Y is a carbonyl, thiocarbonyl, or a 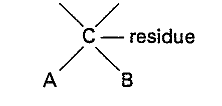 wherein A and B are each severally hydrogen, alkyl, alkylaryl or aryl.

The hydrolysis is carried out in an analogous way, e.g. in the presence of a hydrolysing agent, e.g. in the presence of an acidic agent as e.g. diluted mineral acids, as sulfuric acid or hydrohalogen acid, or in the presence of basic agents as e.g. alkalimetal hydroxides, as sodium hydroxide. Oxycarbonyl residues, aryl sulfonyl residues and cyano groups may in a suitable way be split off by means of acidic agents as by means of a hydrohalogen acid, suitably hydrobromic acid. Preferably the splitting may take place using diluted hydrobromic acid, possibly in a mixture with acetic acid. Cyano groups are preferably split off by means of hydrobromic acid at an elevated temperature, as in boiling hydrobromic acid, according to the "bromo-cyano method" (v. Braun). Further, e.g. a tert.-butoxycarbonyl residue may be split off under anhydrous conditions by means of a treatment with a suitable acid, as trifluor- acetic acid. Acidic agents are preferably used at a hydrolysis of compounds of formula IX.

Residues splitable by ammonolysis are especially the halogencarbonyl residues, as the chlorocarbonyl residue. The ammonolysis may be carried out in a common way, e.g. by means of an amine containing at least one hydrogen atom bound to the nitrogen atom, as a mono- or diloweralkylamino e.g. methylamine or dimethylamine, or especially ammonia, preferably at an elevated temperature. Instead of ammonia one may use an agent which gives ammonia as hexamethylenetetraamine.

Residues splitable by means of a reduction are e.g. an a-arylalkyl residue, as a benzyl residue or an α-aralkoxycarbonyl residue as a benzyloxycarbonyl residue, which in a common way may be split off by means of a hydrogenolysis, especially by catalytically activated hydrogen, as by hydrogen in the presence of hydrogenating catalysts, e.g. Raney-nickel. Further residues splitable by means of hydrogenolysis are 2-halogenalkoxycarbonyl residues as 2,2,2-trichloroethoxycarbonyl residues or 2-iodo- ethoxy- or 2,2,2-tri-bromoethoxycarbonyl residues, which may be split off in a common way, suitably by means of a metallic reduction (so called nascerating hydrogen). Nascerating hydrogen may be obtained by the influence of metal or metal alloys, as amalgam on compounds which give hydrogen as carboxy acids, alcohols or water, whereby especially zink or zinkalloys together with acetic acid may be used. Hydrogenolysis of 2-halogenalkoxycarbonyl residues may further take place using chromium or chromium (II) compounds as chromium (II) chloride or chromium (II) acetate.

A residue splitable by reduction may also be an arylsulfonyl group as a toluenesulfonyl group, which in a common way may be split off by reduction using nascerating hydrogen, e.g. by means of an alkalimetal, as lithium or sodium in liquid ammonia, and suitably may be split off from a nitrogen atom. At the carrying out of the reduction one has to take care of the fact that other reducing groups are not influenced.

Residues splitable by means of pyrolysis, especially residues splitable from the nitrogen atom, are in occurring cases substituted suitably unsubstituted carbamoyl groups. Suitable substituents are e.g. loweralkyl or arylloweralkyl as methyl or benzyl or aryl, as phenyl, the pyrolysis is carried out in a common way, whereby one may have to take care of other thermically susceptible groups.

Residues splitable by means of fermentation, especially residues splitable from the nitrogen atom are in occurring cases substituted, however, suitably unsubstituted carbamoyl groups. Suitable substituents are e.g. loweralkyl or aryl-loweralkyl, as methyl or benzyl, or aryl as phenyl. The fermentation is carried out in a common way, e.g. by means of the enzyme urease or soy bean extract at about 20°C or slightly elevated temperature.

Further, a Schiff's base of formula X or XI 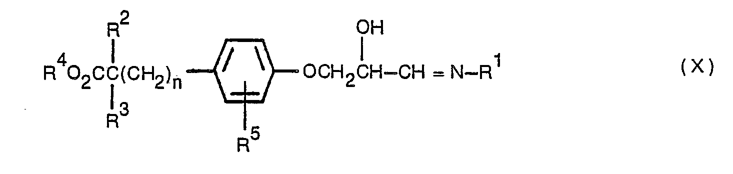 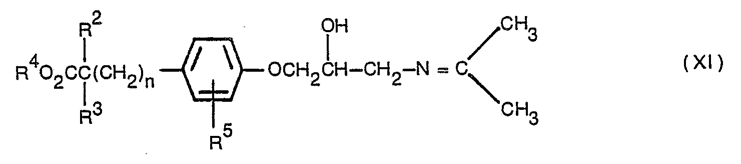 or a cyclic tautomer corresponding to formula XI or formula XII 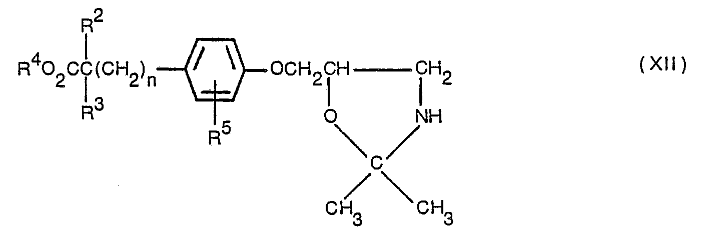 can be reduced, wherein R¹, R², R³, R⁴, R⁵ and n have the same meanings as given above, and whereby the compounds of formula XI and XII may exist together, too.

This reduction is carried out in a common way, e.g. using a di-lightmetalhydride, as sodium- borohydride, lithiumaluminiumhydride, using a hydride as Boran with formic acid, or by means of a catalytic. hydrogenation, as with hydrogen in the presence of Raney-nickel. At the reduction one has to take care of the fact that other groups are not affected.

Further, the oxo group in the compound of formula XIII 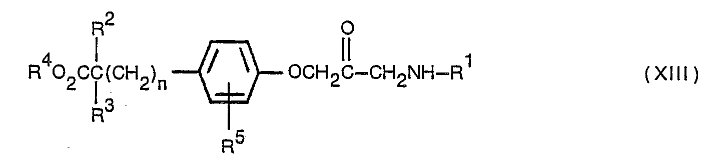 wherein R', R², R³, R⁴, R⁵ and n have the same meanings as given above, can be reduced to a hydroxy group. This reduction is carried out in a common way, especially using a di-lightmetalhydride, as mentioned above, or according to the "Meerwein-Pondorf-Verley method" or a modification thereof, suitably using an alkanol as a reaction component and as solvent, as isopropanol, and using a metal- alkanolate, as metalisopropanolate, e.g. aluminium isopropanolate.

Further, in a compound of formula XIV 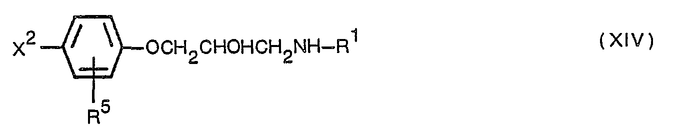 wherein R^{I} and R⁵ have the meanings given above, and X² is a residue, which is able to be transformed to a residue, 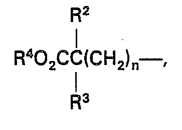 wherein R², R³, R⁴, and n have the meanings given above, one transforms X² to this residue.

X² can thus be a residue of the formula 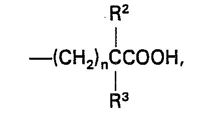 wherein R², R³ and n have the meanings given above, which residue X² is esterified in accordance with processes known per se. Such processes are e.g. esterification in a common way using a strong acid as a catalyst and/or using any form of water removal.

The carboxylic acid can also be esterified by alkylation of the carboxylate in a common way using or not using ion pair technic.

Further the ester group can be formed at the esterification reaction from another functional group, e.g. from a nitril, reacting at these conditions.

The residue 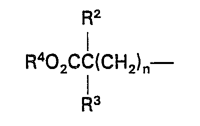 can also be obtained from X² by e.g. reduction of a carbon-carbon unsaturation and/or from another functional group as e.g. a keto group. The desired ester can also be obtained by oxidation of the corresponding ketone.

The residue 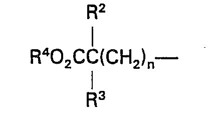 can also be obtained from X² by splitting off, e.g. by hydrogenolysation of a functional group as e.g. a keto, hydroxy, halogen, a group containing a nitrogen atom, and the like. R², R³, R⁴ and n have the meanings given above.

The residue X² can also be of the formula 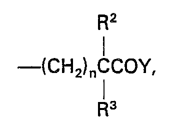 wherein Y is a leaving group such as alkoxy, aryloxy, alkylcarboxy, halogen, etc. The transformation into 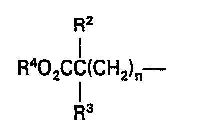 can then take place in different ways i.a. via methods as reesterification, esterification, and the like.

Further, one can react a compound of formula XIV above, wherein X² is -(CH₂)ₙZ, wherein n is an integer 1 or 2, and Z denotes a leaving group as e.g. halogen, or tosyl, with an acetic or propionic acid ester in the presence of a base, e.g. lithium dialkylamide in an aprotic solvent as e.g. tetrahydrofuran.

Thus, the following reactions can take place as examples of the above mentioned reactions, where X² is a group transformable into

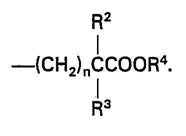

In the following R⁷ is the group 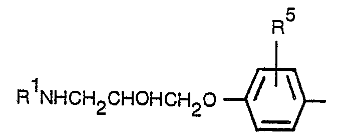 wherein R¹ and R⁵ have the meanings given above:

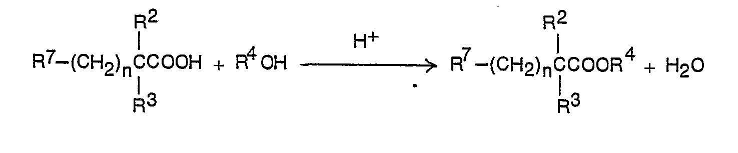

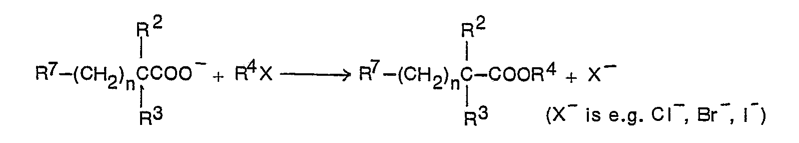

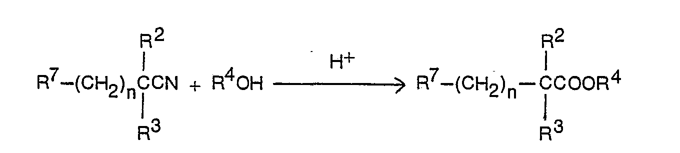

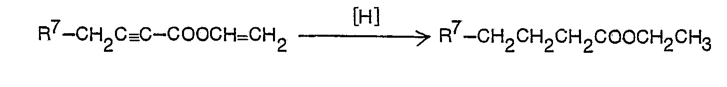

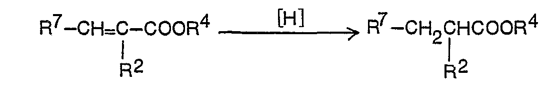

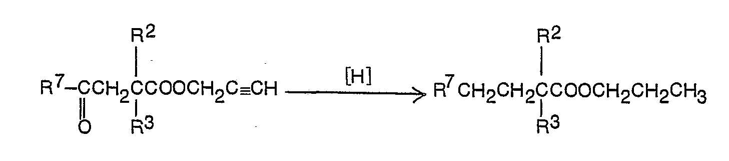

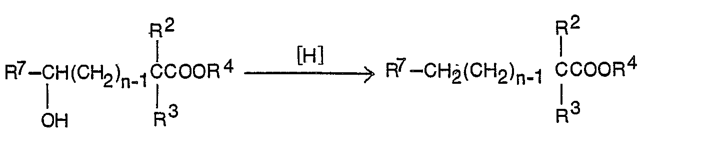

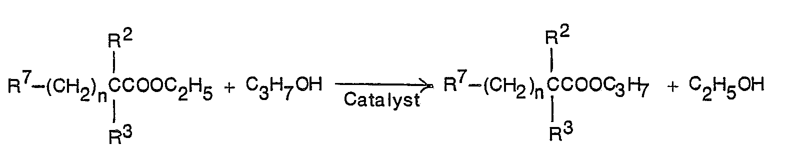

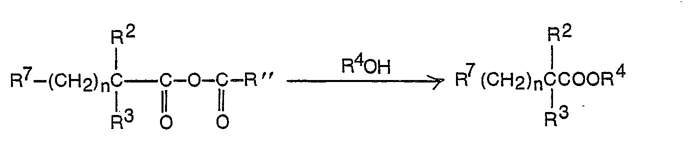

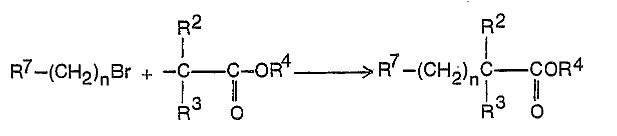

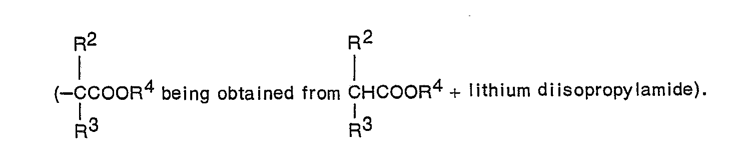

Another method involving reaction and splitting off of a protecting group in the same operation is:

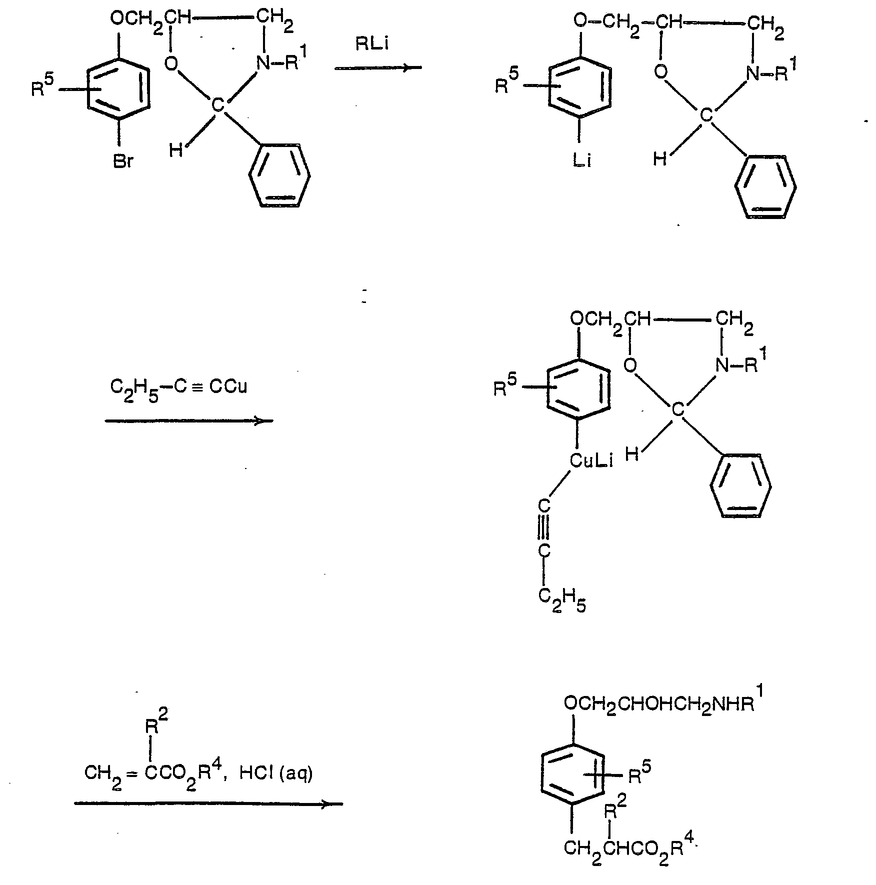

Further, the oxo group in a compound corresponding to these of formula I and which carries an oxo group at a carbon atom bound to a nitrogen atom may be reduced by two hydrogen atoms.

Said compounds are e.g. such of the formula XV 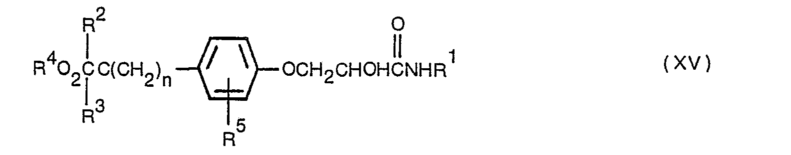 wherein R and n have the meanings as given above.

The reduction can be carried out according to the above described manner using complex metal- hydrides, e.g. lithiumaluminiumhydride or di-isobutylaluminiumhydride. Suitably the reaction takes place in an inert solvent as an ether, e.g. diethylether or tetrahydrofuran. Complex borohydrides can also be used especially when a selective reaction is wanted.

Further, a compound of the present invention can be obtained by catalytic hydrogenation of a compound of the invention containing one or more substituents in the aromatic ring that can be split off by means of catalytic hydrogenation. Such substituents are F, Br, CI and I. The splitting off can also take place in connection with prior mentioned methods.

Depending on the process conditions and the starting material the end product is obtained either in free form or in the form of its acid addition salt, which is included in the scope of the invention. Thus, for example, basic, neutral or mixed salts may be obtained as well as hemiamino, sesqui- or poly- hydrates. The acid addition salts of the new compounds may in a manner known per se be transformed into free compounds using e.g. basic agents as alkali or ion exchanger. On the other hand, the free bases obtained may form salts with organic or inorganic acids. In the preparation of acid addition salts preferably such acids are used which form suitable therapeutically acceptable salts. Such acids are e.g. hydrohalogen acids, sulfuric acid, phosphoric acid, nitric acid, perchloric acid, aliphatic, alicyclic, aromatic or heterocyclic, carboxy or sulfonic acids, as formic, acetic, propionic, succinic, glycolic, lactic, malic, tartaric, citric, ascorbic, maleic, hydroxymaleic or pyruvic acid, phenylacetic, benzoic, p-aminobenzoic, anthranilic, p-hydroxybenzoic, salicyclic or p-aminosalicyclic acid, embonic acid, methanesulfonic, ethanesulfonic, hydroxyethanesulfonic, ethylenesulfonic acids, halogenbenzenesulfonic, toluenesulfonic, naphthylsulphonic acids, or sulfanilic acid; methionine, tryptophane, lysine or arginine.

These or other salts of the new compounds as e.g. picrates may serve as purifying agents or the free bases obtained as the free bases are transformed into salts, these are separated and the bases are then set free from the salts again. According to the close relationship between the new compounds in free form and in the form of their salts it will be understood from the above and the below that, if possible, the corresponding salts are included in the free compound.

The invention also relates to any embodiment of the process of which one starts from any compound obtained as an intermediate in any process step and one carries out the lacking process step, or one breaks off the process at any step, or at which one forms a starting material under the reaction conditions, or at which a reaction component possibly in the form of its salt is present.

Thus, one may react an aldehyde of the formula XVI 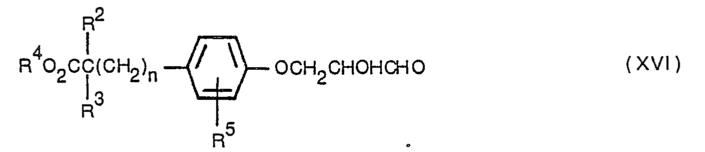 wherein R², R³, R⁴, R⁵ and n have the same meanings as given above, with an amine of the formula XVII 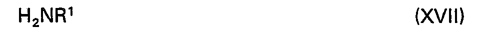 wherein .RI has the meaning given above in the presence of a suitable reducing agent, as one of the above mentioned. Thereby a compound of formula X is obtained as an intermediate, which then is reduced according to the invention.

Further, one may in a manner known per se react an amine of the formula IV with a keton of the formula XVIII 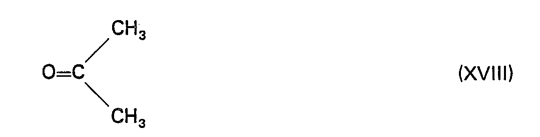 in the presence of a suitable reducing agent, as one of the above mentioned to produce compounds of formula XI or XII as an intermediate, which then is reduced according to the invention.

Further, in a compound of the formula XIX 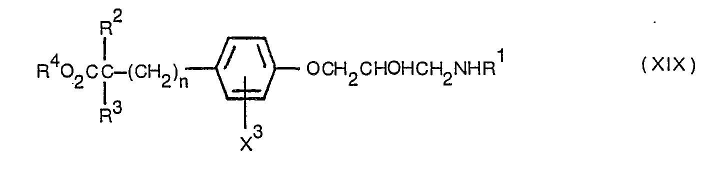 wherein R', R², R³, R⁴, and n have the same meanings as given above, and X³ is a residue transformable into R⁵, X³ is transformed into R⁵.

X³ can thus be a carbon-carbon unsaturated residue and/or a carbonyl containing residue which residues are hydrogenated to give a residue R⁵. Thus an alkynyl or an alkynyloxy group can be hydrogenated to give an alkenyl group, or an alkenyloxy group, respectively, or an alkyl group, or an alkoxy group respectively, depending on how far the hydrogenation is carried out.

Further X³ can be e.g. a residue 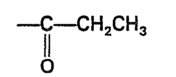 which is hydrogenated to give an alkyl R⁵.

Further X³ can be a residue -OH which is esterified in a known way to give an alkoxy, alkenyloxy, or alkynyloxy group _{R}⁵.

Further, X³ can be hydrogen, whereby R⁵ being halogen, preferably chloro and bromo can be added.

Thus, a compound of the formula 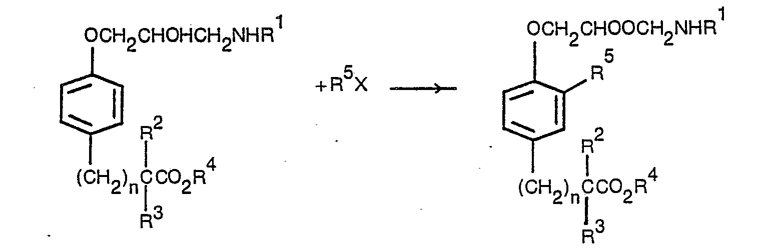 whereby X is halogen or R⁵X is, e.g. tert.butylhypochlorite.

The new compounds may, depending on the choice of staring materials and process, be present as optical antipodes or racemate, or, if they contain at least two asymmetric carbon atoms, be present as an isomer mixture (racemate mixture).

The isomer mixtures (racemate mixtures) obtained may depending on physical-chemical differences of the component, be separated into the both stereoisomeric (diastereomeric) pure racemate e.g. by means of chromatography and/or fractionated crystallization.

The racemates obtained can be separated according to known methods, e.g. by means of recrystallization from an optical active solvent, by means of microorganisms, or by a reaction with optically active acids forming salts of the compound and separating the salts thus obtained, e.g. by means of their different solubility in the diastereomers, from which the antipodes by the influence of a suitable agent may be set free.

Suitably useable optically active acids are e.g. the L- and D-forms of tartaric acid, di-o-tolyl- tartaric acid, malic acid, mandelic acid, camphersulfonic acid or china acid. Preferably the more active part of the two antipodes is isolated. Further one of the two enantiomers can be obtained by asymmetrical reduction of the corresponding Keto-compound.

Suitably such starting materials are used for carrying out the reactions of the invention, which material leads to groups of end products primarily especially desired and especially to the specifically described and preferred end products.

The starting materials are known or may, if they should be new, be obtained according to processes known per se.

In clinical use the compounds of the invention are administered normally by injection in the form of a pharmaceutical preparation, which contains an active component either as free base or as pharmaceutically acceptable, non-toxic acid addition salts, e.g. the hydrochloride, lactate, acetate, sulphamate or the like in combination with a pharmaceutical carrier.

Thereby the mentioning of the new compounds of the invention is here related to either the free amine base or the acid addition salts of the free base, even if the compounds are generally or specifically described, provided that the context in which such expressions are used, e.g. in the examples, with this broad meaning should not correspond. The carrier may be a liquid diluent or a capsule. These pharmaceutical preparations are a further object of the invention. Usually the amount of active compound is between 0.1 to 99% by weight of the preparation, suitably between 0.5 to 20% by weight in preparations for injection.

Solutions for parenteral administration by injection may be prepared as an aqueous solution of a watersoluble pharmaceutically acceptable salt of the active compound, preferably in a concentration from about 0.5% by weight to about 20% by weight. These solutions may also contain stabilizing agents and/or buffering agents and may suitably be available in different dosage unit ampoules.

The daily dose of the active substance varies and is depending on the acceptance but as a general rule it is 1-100 mg/min of active substance at intravenous administration, (average weight human), more particularly 15-45 mg/min.

### Best mode of carrying out the invention

The following illustrates the principle and the adaption of the invention, however, without being limited thereto. Temperature is given in degrees Celsius.

### Example 1

### Preparation of methyl 3-[4-(2-hydroxy-3.-isopropylaminopropoxy)phenyl]propanoate (Method a)

4.5 g of methyl 3-[4-(2,3-epoxypropoxy)phenyl]-propanoate, 15 ml of isopropylamine, and 25 ml of isopropanol were heated on a steam bath in an autoclave for 5 hours. The volatile compounds were evaporated in vacuo and the residue thus obtained was dissolved in ethylacetate and treated with sulphuric acid. The neutral sulphate of methyl 3-[4-(2-hydroxy-3-isopropylamino propoxy)phenyl]-propanoate was thus formed. Yield 1.8 g. Melting point 164°C. The structure was determined using NMR and equivalent weight.

### Example 2

Ethyl 3-[4-(2-hydroxy-3-isopropylaminopropoxy)-phenyl]propanoate was prepared in accordance with Example 1 using 7.0 g of ethyl 3-[4-(2,3-epoxypropoxy)phenyl]propanoate and 20 ml of isopropylamine as starting materials. Melting point 149°C (1/2 H_{Z}S0₄). Yield 2.8 g. The structure was determined by NMR and equivalent weight.

### Example 3

2-Methoxyethyl 3-[4-(2-hydroxy-3-isopropylaminopropoxy)phenyl]propanoate was prepared in accordance with Example 1 above, using 25 ml of isopropylamine and 8.0 g of 2-methoxyethyl 3-[4-(2,3-epoxypropoxy)phenyl]propanoate as starting materials. Melting point 98°C (p-HO-benzoate). Yield 2.0 g. The structure was determined by NMR and equivalent weight.

### Example 4

2-Methoxyethyl 4-[4-(2-hydroxy-3-isopropylaminopropoxy)phenyl]butanoate was prepared in accordance with Example 1 above, using 30 ml of isopropylamine and 12.1 g of 2-methoxyethyl 4-[4-(2,3-epoxypropoxy)phenyl]butanoate as starting materials. Melting point 90°C (p-HO-benzoate). Yield 4.1 g. The structure was determined by NMR and equivalent weight.

### Example 5

Propyl 3-[4-(2-hydroxy-3-isopropylaminopropoxy)phenyl]propanoate was prepared in accordance with Example 1 above using 9.0 ml of isopropylamine and 3.2 g of propyl 3-[4-(2,3-epoxy- propoxy)-phenyl]propanoate as starting materials. Melting point 100°C (HCI). Yield 1.4 g. The structure was determined by NMR and equivalent weight.

### Example 6

Isopropyl 3-[4-(2-hydroxy-3-isopropylaminopropoxy)phenyl]propanoate was prepared in accordance with Example 1 above, using 10 ml of isopropylamine and 5.0 g of isopropyl 3-[4-(2,3-epoxypropoxy)phenyl]propanoate as starting materials. Melting point 97°C (HCI). Yield 3.5 g. The structure was determined by NMR and equivalent weight.

### Example 7

Ethyl 4-[4-(2-hydroxy-3-isopropylaminopropoxy)phenyl]butanoate was prepared in accordance with Example 1 above using 50 ml of isopropylamine and 13.9 g of ethyl-4-[4-(2,3-epoxypropoxy)-phenyl]butanoate as starting materials. Melting point 85°C (p-HO-benzoate. Yield 2.0 g. The structure was determined by NMR and equivalent weight.

### Example 8

Ethyl 4-[3-bromo-4-(2-hydroxy-3-isopropylaminopropoxy)phenyl]butanoate was prepared in accordance with Example 1 above, using 12.5 ml of isopropylamine and 6.0 g of ethyl 4-[3-bromo-4-(2,3-epoxypropoxy)phenyl]butanoate as starting materials. Melting point 110°C (HCI). Yield 3.7 g. The structure was determined by NMR and equivalent weight.

### Example 9 (method b)

10 g of ethyl 4-[4-(2,3-epoxypropoxy)phenyl]butanoate in 100 ml of ethanol were saturated with gaseous ammonia and the mixture was heated in an autoclave on a boiling waterbath for 4 hours. The solvent was evaporated and the residue was dissolved in ethylacetate and HCI-gas was introduced. The hydrochloride then precipitated was filtered off and dissolved in 50 ml of ethanol to which 2-bromopropane and 10.5 kg K₂C0₃ had been added. The mixture was refluxed for 10 hours and filtered whereupon the solvent was evaporated. The residue was dissolved in ethylacetate. The p-HO-benzoate of ethyl 4-[4-(2-hydroxy-3-isopropylaminopropoxy)phenyl]butanoate was precipitated by introducing an equivalent amount of p-HO-benzoic acid. Melting point 85°C.

### Example 10 (method c)

2.4 g of Na were dissolved in 100 ml of ethanol, whereupon 20.8 g of ethyl 4-[4-hydroxyphenyl]-butanoate and 13.9 g of 1-chloro-3-isopropylaminopropanol-2 were added. The mixture was heated in an autoclave on a boiling water-bath for 10 hours. Thereupon it was filtered and the filtrate was evaporated to dryness. The residue was made acid to pH 3 using HCI and extracted with ether, whereupon the aqueous phase was made alkaline with ammonia and extracted with ethylacetate. The ethylacetate phase was dried over MgS0₄ and filtered. the p-HO-benzoate of ethyl 4-[4-(2-hydroxy-3-isopropyl- aminopropoxy)phenyl]butanoate was precipitated using an equivalent amount of p-HO-benzoic acid. Melting point 85°C.

### Example 11 (method d)

0.12 moles of ethyl 4-[4-hydroxyphenyl]butanoate were mixed with 0.080 moles of 1-isopropyl-3-acetidinol, 0.500 moles of benzylalcohol and 0.002 moles of KOH. The mixture was refluxed while stirring for 6 hours at 140°C and was then cooled and extracted with 1 N HCI. The aqueous phase was made alkaline, and was finally extracted with ethylacetate. After drying over MgS0₄ and filtering the p-HO-benzoate of ethyl 4-[4-(2-hydroxy-3-isopropylaminopropoxy)phenyl]-butanoate was precipitated using an equivalent amount of p-HO-benzoic acid. Melting point 85°C.

### Example 12 (method f)

In accordance with Example 10 above ethyl 4-[4-(3-amino-2-hydroxypropoxy)phenyl]butanoate was prepared. 2.8 g of this compound were dissolved in 30 ml of methanol and excess amount of acetone was added, whereby ethyl 4-[4-(2-hydroxy-3-isopropyliminopropoxy)-phenyl]butanoate was obtained. The solvents were evaporated and a new solution was obtained by adding 30 ml of methanol. The solution was cooled to 0°C and at this temperature 3 g of sodium borohydride were added little by little, whereby the imino compound was reduced. The temperature was then allowed to rise to ambient temperature and after 1 hour 100 ml of H₂₀ were added and the total mixture was extracted with ethylacetate. The ethylacetate phase was dried over M_{g}SO₄ and filtered. The residue was transformed into its p-HO-benzoate. In this way ethyl 4-[4-(2-hydroxy-3-isopropylaminopropoxy)phenyl butanoate p-HO-benzoate was obtained. Melting point 85°C.

### Example 13 (method g)

1.0 g of ethyl 4-[4-(3-isopropylamino-2-oxo-propoxy)-phenyllbutanoate was dissolved in 25 mls of methanol and the solution was cooled to 0°C on an ice-bath. 0.25 g of NaBH₄ were added little by little while stirring first at 0°C for 1 hour and then at ambient temperature for 0.5 hour. The solution thus obtained was evaporated whereupon 50 ml of H₂0 were added. The aqueous phase was extracted 3 times with ethylacetate. The collected ethylacetate phase was dried over MgS0₄ and filtered. The p-HO-benzoate was precipitated by introducing an equivalent amount of p-HO-benzoic acid. The p-HO-benzoate of ethyl 4-[4-(2-hydroxy-3-isopropylaminopropoxy)phenyl]butanoate melted at 85°C.

### Example 14 (method c + h)

2.0 g of ethyl 4-[4-[3-(N-benzylisopropylamino)2-hydroxypropoxyl-phenyll-3-butenoate were dissolved in 100 ml of ethanol. 0.2 g of Pd/C (5%)-catalyst were added and the mixture was hydrogenated until 230 mls of hydrogen had been absorbed. The catalyst was filtered off and the residue was evaporated. The residue thus obtained was dissolved in ethylacetate and the ethylacetate phase was treated with an equivalent amount of p-HO-benzoic acid, whereby the p-HO-benzoate of ethyl 4-[4-(2-hydroxy-3-isopropylaminopropoxy)phenyl]butanoate precipitated. Melting point 85°C

### Example 15 (method h)

7.1 g of the hydrochloride of methyl 3-[4-(hydroxy-3-isopropylaminopropoxy)-3-methoxy- phenyllpropanoate was dissolved in 400 ml of ethanol and hydrogenated over Pd/C. The reaction stopped when 500 ml of hydrogen had been absorbed. The catalyst was filtered off and the solvent evaporated. The residue was treated with ether and the hydrochloride of methyl 3-[4-(2-hydroxy-3-isopropylaminopropoxy)-3-methoxyphenyl]propanoate crystallized. Yield 6.4 g. Melting point 95°C (HCI). The structure was determined using NMR, IR, and equivalent weight.

### Example 16 (method h)

2.0 g of 2-[4-(2-hydroxy-3-isopropylaminopropoxy)phenyl]propanic acid were dissolved in 50 ml of propanol. The solution was saturated with gaseous HCI and refluxed for 2 hours. The excess of propanol was evaporated in vacuo and the residue was crystallized from ethylacetate. The hydrochloride of propyl 3-[4-(2-hydroxy-3-isopropylaminopropoxy)phenyl]propanoate melted at 100°C.

### Example 17 (method h)

2.0 g of methyl 3-[4-(2-hydroxy-3-isopropylaminopropoxy)phenyl]propanoate were dissolved in 100 mls of propanol. The solution was treated with gaseous HCI and refluxed over night. After evaporation of excess of alkanols the hydrochloride of propyl 3-[4-(2-hydroxy-3-isopropylaminopropoxy)-phenyl]propanoate was obtained. Melting point 100°C.

### Example 18 (method h)

2.0 g of propyl 3-[4-(2-hydroxy-3-isopropylaminopropoxy)phenyl]3-oxo-propanoate were mixed with 10 ml of triethyleneglycol and 0.35 ml of 85% hydrazine hydrate. The mixture was refluxed for 1.5 hours. Thereupon so much of water was distilled off that the temperature could be raised to 200°C. The mixture was then boiled at 200°C for 4 hours. After cooling to ambient temperature 10 mls of water and 6.5 ml of 2N HCI were added. The solution was extracted with chloroform, dried over MgS0₄ and evaporated in vacuo. The residue was crystallized from ethylacetate. The hydrochloride of propyl 3-[4-(2-hydroxy-3-isopropylaminopropoxy)phenyl]propanoate melted at 100°C.

### Example 19 (method h)

1.76 g of ethyl acetate dissolved in 50 ml of tetrahydrofuran were treated with 1.3 g of lithium- diisopropylamide at -78°C. After 10 min 2.5 g of 1-(4-chloromethylphenoxy)-3-isopropylaminopropanol-2 were added. The total mixture was allowed to raise its temperature to ambient temperature, whereupon the volatite components were evaporated in vacuo. The residue was treated with ethyl acetate and after filtration the neutral sulphate of ethyl 3-[4-(2-hydroxy-3-isopropylamino- propoxy)phenyl]propanoate was precipitated using sulphuric acid. Melting point 149°C.

### Example 20

### Preparation of ethyl 3-[3-bromo-4-(2-hydroxy-3-isopropylaminopropoxy)phenyl]propanoate (method a)

This compound was prepared in accordance with Example 1 above using 11.9 g of ethyl 3-[3-bromo-4-(2,3-epoxypropoxy)phenyl]propanoate and 30 ml of isopropylamino as starting materials. Melting point 117°C (HCI). Yield 8.0 g. The structure was determined by NMR.

### Example 21

### Preparation of ethyl 3-[4-(2-hydroxy-3-isopropylaminopropoxy)-3-methoxyphenyl]propanoate (method h)

This compound was prepared by dissolving ethyl 3-[4-(2-hydroxy-3-isopropylaminopropoxy)-3-methoxyphenyl]propenate hydrochloride in 50 ml of ethanol, adding 0.5 g of 5% Pd/C catalyst and hydrogenating the mixture at NTP 240 ml of hydrogen were consumed. The catalyst was then filtered off and the solvent evaporated. The residue was treated with ether and the hydrochloride of the compound precipitated. Yield 2.4 g. Melting point 69-71 °C. The structure was determined using NMR.

### Example 22

### Preparation of ethyl 3-[3-ethoxy-4-(2-hydroxy-3-isopropylaminopropoxy)phenyl]propanoate (method h)

This compound was prepared in accordance with Example 20 above using 7.0 g of ethyl 3-[3- ethoxy-4-(2-hydroxy-3-isopropylaminopropoxy)phenyl]propenoate as starting material. Yield 4.8 g. Melting point 82°C (HCI). The structure was determined by NMR.

### Example 23

### Preparation of ethyl 3-[3-chloro-4-(2-hydroxy-3-isopropylaminopropoxy)phenyl]propanoate

This compound was prepared in accordance with Example 1 above using 10.5 g of ethyl-3-[3-chloro-4-(2,3-epoxypropoxy)phenyl]propanoate and 50 ml of isopropylamine as starting materials. Melting point 93-94°C (HCI). Yield 4.0 g. The structure was determined by NMR and equivalent weight.

### Example 24

### Preparation of isopropyl 3-[4-(2-hydroxy-3-isopropylaminopropoxy)-3-methoxyphenyl]propanoate

This compound was prepared in accordance with Example 20 above using 4.3 g of isopropyl 3-[4-(2-hydroxy-3-isopropylaminopropoxy)-3-methoxyphenyl]propenaote as. starting material. Yield 2.4 g. Melting point: oil (1/2 H₂SO₄). The structure was determined using NMR. Refraction index: n', = 1.516.

### Example 25

### Preparation of ethyl 3-[3-fluoro-4-(2-hydroxy-3-isopropylaminopropoxy)phenyl]propanoate

This compound was prepared in accordance with Example 1 above using 15.0 g of ethyl 3-[3-(fluoro-4-(2,3-epoxypropoxy)phenyl]propanoate and 100 ml of isopropylamine as starting materials. Melting point81°C (HCI). Yield 5.0 g. The structure was determined using NMR and equivalent weight.

### Example 26

Ethyl 3-[3-fluoro-4-(2-hydroxy-3-tert.butylaminopropoxy)phenyl]propanoate was prepared in accordance with Example 1 above using 13.0 g of ethyl 3-[3-fluoro-4-(2,3-epoxypropoxy)phenyl]propanoate and 20 ml of tert.butylamine as starting materials. Melting point 118°C (HCI). The structure was determined by NMR.

### Example 27

Ethyl 3-[3-bromo-4-(2-hydroxy-3-isopropylaminopropoxy)phenyl]propanoate hydrochloride (5 g) was dissolved in 20 ml of ethanol. The solution was sterile filtered and filled into ampoules. This solution is diluted several times its volume with a physiologically acceptable aqueous solution before use.

### Example 28

Ethyl 3-[3-chloro-4-(2-hydroxy-3-isopropylaminopropoxy)phenyllpropanoate hydrochloride (10 g) was dissolved in 100 ml of water and sterile filtered. This solution was filled into ampounes which were freeze dried and sealed under sterile conditions. The ampoules are used for preparing infusion solutions by dissolving their contents in a physiologically acceptable aqueous solution.

### Biological effects

The beta-adrenoceptor blocking agents of the present invention were tested as regards their biological properties. All compounds were thereby tested in two different experimental models but with the same species, premedication and preparation. Cats (males and females weighing 2.5-3.5 kg) were pretreated with reserpine (5 mg/kg bodyweight administered intraperitoneally) about 16 hours before the experiments. The animals were pretreated with reserpine in order to eliminate the endogenous sympathetic control of heart rate and vascular smooth muscle tone. The cats were anaesthetized with pentobarbital (30 mg/kg bodyweight administered i.p.) and artificially ventilated with room air. A bilateral vagotomy was performed in the neck. Blood pressure was obtained from a cannulated carotid artery and heart rate was recorded from a cardiotachometer, triggered by the oscillations in blood pressure. The femoral artery of one hind leg was cannulated in both directions. Blood taken from the proximal part was pumped, at a constant flowrate, back into the distal part of the artery by means of a roller pump (Watson-Marlow). The perfusion pressure (PP) was recorded. Changes in PP indicated changes in the peripheral vascular resistance of the leg.

### Experiment A

The maximal heart rate and vasodilator responses to isoprenaline were established by injecting a high i.v. bolus dose of isoprenaline (2.0 µmol/kg). An isoprenaline infusion rate was then established which gave a heart rate response (at steady state) which was about 80% of the maximal heart rate response to isoprenaline. Usually this dose of isoprenaline was 0.25 µmol/kg x min. The vasodilator response to this dose of isoprenaline was also approximately 80% of maximal. The isoprenaline dose was then infused during periods of 20 minutes with an interval of 20 minutes between the infusion periods.

The test compound was administered as an intravenous bolus injection seven minutes after start of each isoprenaline infusion. The dose of the test compound was increased until total blockade of the isoprenaline responses was achieved.

For each dose of the test compound the peak reduction of the isoprenaline heart rate response was expressed as per cent blockade according to the formula:

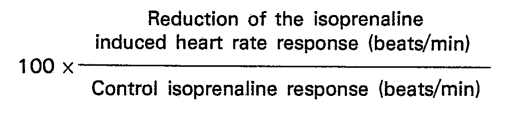

Per cent blockade (for each dose) was then plotted against log dose of the test compound. The ED:50 value (i.e. the dose which produced half maximal blockade) was interpolated.

The plasma half life was estimated by the method of Levy (Nagashima, R., O'Reilly, R. A. and Levy, G., Clin. Pharmacol. Ther., 10 (1969) 22). The time from the peak inhibitory effect of each dose until 50% recovery was determined and plotted against log dose of the compound. The slope of the line obtained was calculated with linear regression. The slope equals 2.303/K_{E} where Kₑ is the rate constant for elimination. The plasma half life (t 1/2) was then calculated according to the relation t 1/2 = 0.693/K_{E}.

### Experiment B

The maximal heart rate and vasodilator responses to isoprenaline were established by injecting a high i.v. bolus dose of isoprenaline (2.0 µmol/kg). An isoprenaline bolus dose which gave a heart rate response which was about 80% of the maximal response was then tested out. Usually this dose of isoprenaline was 0.4 µmol/kg. The vasodilator response to this dose of isoprenaline was also approxi- ₅ mately 80% of maximal.

The test compound was then infused intravenously in increasing doses. Each dose was given during 12 minutes with an interval of 18 minutes between doses.

The control dose of isoprenaline was injected 10 minutes after start of each infusion of the test compound. The dose of the test compound was increased logarithmically until total blockade of the isoprenaline responses was achieved.

The inhibitory effect of each dose of the test compound was expressed as per cent blockade according to the formula:

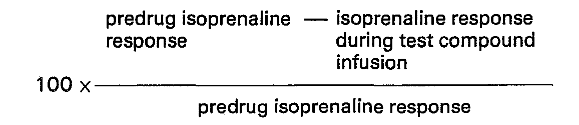

Per cent blockade was then plotted against log dose of the blocker and the ED:50-value was interpolated (cf. above). The ED:50 for heart rate blockade and ED:50 for blockade of the vaso- dilatation could then be compared and the selectivity for the compound estimated. Intrinsic sympa- thomimetic activity was evaluated as the maximal heart rate elevation during infusion of the test compound.

The experiments demonstrate that the compounds of the invention are potent beta-adrenoceptor antagonists with a higher potency as regards blockade of the cardiac beta-adrenoceptors than of vascular beta-adrenoceptors. Furthermore, the estimated plasma half lifes of the compounds are shorter than ten minutes.

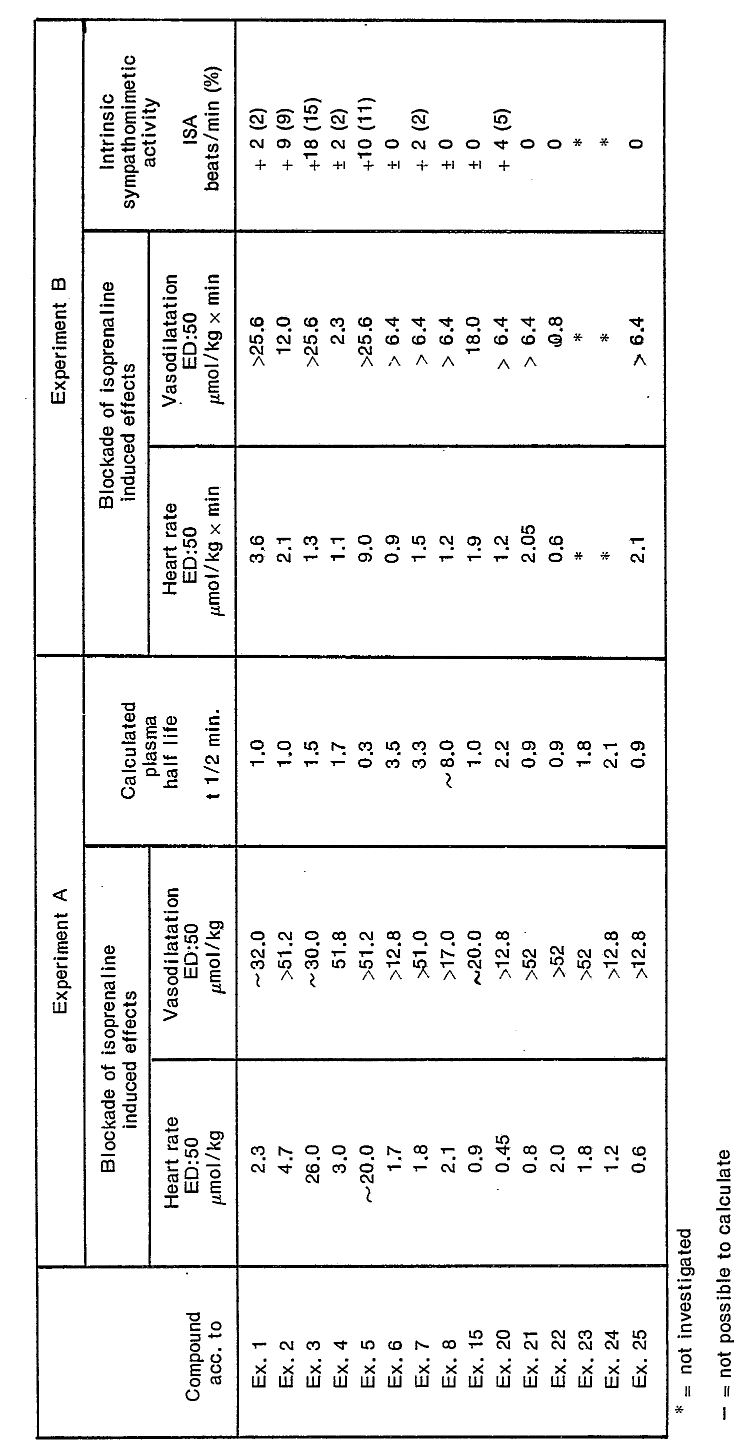

## Claims (Claims for the following Contracting State(s) : s: BE CH DE FR GB IT LI LU NL SE)

1. A compound of the formula I 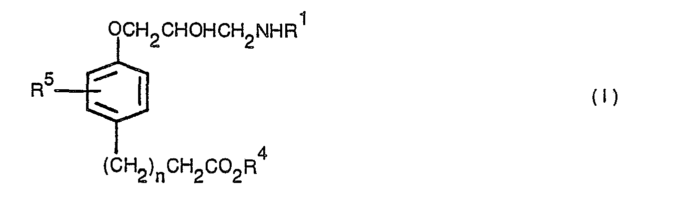 wherein R^{I} is selected from the group consisting of isopropyl and tert. butyl, R⁵ is selected from the group consisting of hydrogen, halogen, lower alkyl having up to 4 carbon atoms, lower alkenyl having up to 4 carbon atoms, and lower alkoxy having up to 4 carbon atoms, R⁴ is selected from the group consisting of alkyl having up to 7 carbon atoms, alkoxyalkyl having up to 8 carbon atoms, cycloalkyl having 3-6 carbon atoms, phenyl, arylalkyl having up to 10 carbon atoms, and hydroxyalkyl having up to 7 carbon atoms, and n is an integer 1 or 2, or a therapeutically acceptable salt of such a compound.

2. A compound according to claim 1, wherein R^{I} is selected from the group consisting of iisopropyl and tert. butyl, R⁵ is selected from the group consisting of hydrogen, halogen, alkyl having up to 4 carbon atoms, alkenyl having up to 4 carbon atoms, and alkoxy having up to 4 carbon atoms, R⁴ is selected from the group consisting of alkyl having up to 7 carbon atoms, alkoxyalkyl having up to 8 carbon atoms, cycloalkyl having 3 to 6 carbon atoms, arylalkyl having up to 10 carbon atoms and hydroxyalkyl having up to 7 carbon atoms, and n is an integer 1 or 2, or a therapeutically acceptable salt of such a compound.

3. Methyl 3-[4-(2-hydroxy-3-isopropylaminopropoxy)phenyl]propanoate, or a therapeutically acceptable salt thereof.

4. Ethyl 3-[4-(2-hydroxy-3-isopropylaminopropoxy)phenyl]propanoate, or a therapeutically acceptable salt thereof.

5. Methyl 3-[4-(2-hydroxy-3-isopropylaminopropoxy)-3-methoxyphenyl]-propanoate, or a therapeutically acceptable salt thereof.

6. 2-Methoxyethyl 4-[4-(2-hydroxy-3-isopropylaminopropoxy)phenyl]-butanoate, or a therapeutically acceptable salt thereof.

7. Ethyl 3-[3-chloro-4-(2-hydroxy-3-isopropylaminopropoxy)phenyl]-propanoate, or a therapeutically acceptable salt thereof.

8. Ethyl 3-[3-fluoro-4-(2-hydroxy-3-isopropylaminopropoxy)phenyl]-propanoate, or a therapeutically acceptable salt thereof.

9. Ethyl 3-[4-(2-hydroxy-3-isopropylaminopropoxy)-3-methoxyphenyl]-propanoate, or a therapeutically acceptable salt thereof.

10. Ethyl 3-[3-ethoxy-4-(2-hydroxy-3-isopropylaminopropoxy)phenyl]-propanoate, or a therapeutically acceptable salt thereof.

11. Process for the preparation of a new amine compound of the formula I 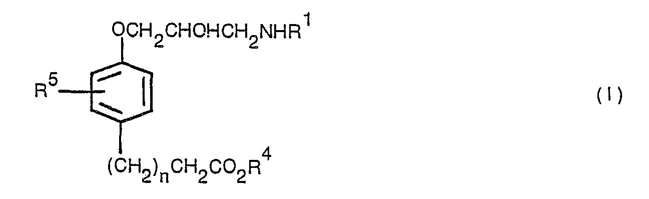 wherein R' is selected from the group consisting of isopropyl and tert. butyl, R⁵ is selected from the group consisting of hydrogen, halogen, lower alkyl having up to 4 carbon atoms, lower alkenyl having up to 4 carbon atoms, and lower alkoxy having up to 4 carbon atoms, R⁴ is selected from the group consisting of alkyl having up to 7 carbon atoms, alkoxyalkyl having up to 8 carbon atoms, cycloalkyl having 3-6 carbon atoms, phenyl, arylalkyl having up to 10 carbon atoms, and hydroxyalkyl having up to 7 carbon atoms, and n is an integer 1 or 2, or a therapeutically acceptable salt thereof, characterized in that
a) a compound of the formula II 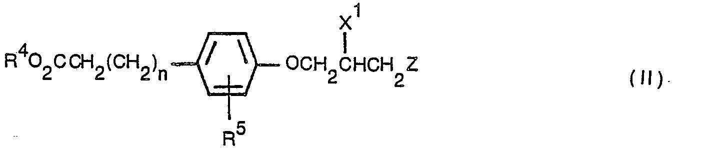 wherein R⁴, R⁵ and n have the same meanings as given above and X¹ is a hydroxy group and Z is a reactive, esterified hydroxy group, or X¹ and Z together form an epoxy group, is reacted with an amine of the formula III 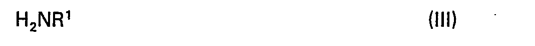 wherein R¹ has the meaning given above:
b) a compound of the formula IV 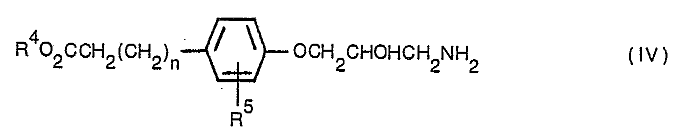 wherein R⁴, R⁵ and n have the same meanings as given above, is reacted with a compound of the formula V 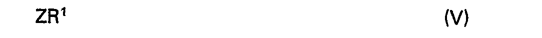 wherein R¹ and Z have the same meanings as given above; or
c) a compound of the formula VI 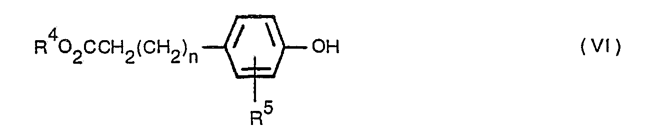 wherein R⁴, R⁵ and n have the same meanings as given above, is reacted with a compound of formula VII 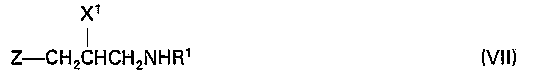 wherein R¹ and X¹ have the same meanings as given above; or
d) a compound of the formula VI 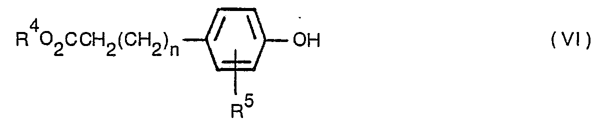 wherein R⁴, R⁵ and n have the same meanings as given above, is reacted with a compound of the formula VIII 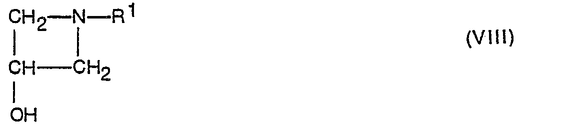 wherein R¹ has the same meaning as given above;
e) from a compound of the formula I, wherein R¹, R⁴, R⁵ and n have the same meanings as above, and which compound has a splitable residue at the nitrogen atom of the amino group and/or has a splitable residue at the hydroxy groups, this residue is split off; or
f) a Schiff's base of the formula X or XI 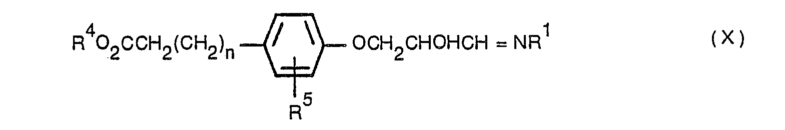 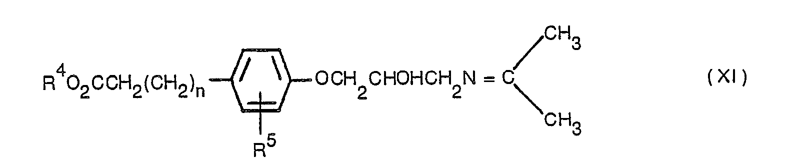 or a cyclic tautomer of formula XII corresponding to the compound of formula XI 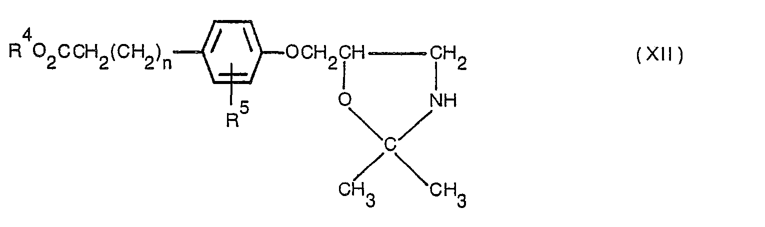 'wherein R¹, R⁴, R⁵ and n have the same meanings as above, whereby the compounds XI and XII may be present simultaneously, is reduced, or
g) in a compound of the formula XIII 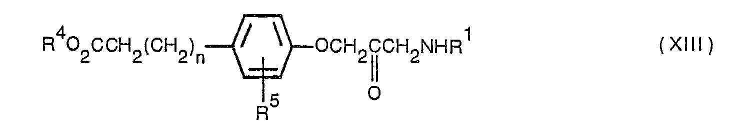 wherein R¹, R⁴, R⁵ and n have the same meanings as above, the oxo group is reduced to a hydroxy group,
h) in a compound of the formula XIV 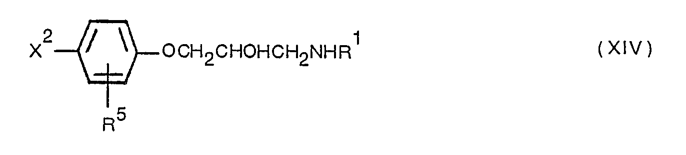 wherein R¹ and R⁵ have the same meanings as given above, X² is a residue transformable into R⁴O₂CCH₂(CH₂)ₙ- wherein R⁴ and n have the above meanings, X² is transformed into R₄O₂CCH₂(CH₂)ₙ-, or
i) reducing a compound of formula XV 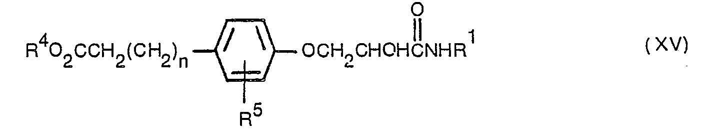 wherein R¹, R⁴, R⁵ and n have the same meanings as above; or
k) in a compound of the formula XIX 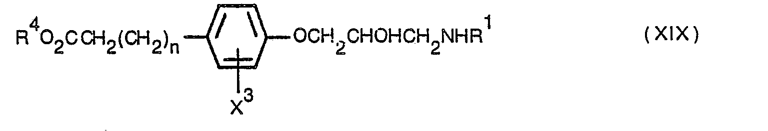 wherein R¹, R⁴ and n have the same meanings as given above, and X³ is a residue transformable into R⁵, X³ is transformed into R⁵;
I) hydrogenating a compound of the formula 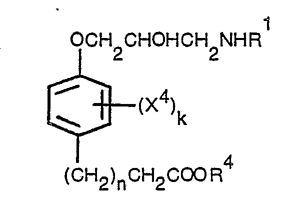 wherein R', R⁴ and n have the meanings given above, and X⁴ denotes a residue that can be split off, and k is an integer 1 to 4, to split X³ off, to the formation of compounds wherein R⁵ is hydrogen; and if desired, substituents are introduced, split off, or reacted in the compounds of the definition of the end product, or compounds obtained are transformed into other end products and/or isomer mixtures obtained are separated into pure isomers, and/or racemates obtained are separated into optical antipodes and/or free bases obtained are transformed into their therapeutically acceptable salt or salts obtained are transformed into their free bases.

12. A pharmaceutical preparation intended for intravenous administration for the treatment of acute myocardial infarction in a mammal, which preparation comprises at least one A-receptor blocking phenoxy-hydroxypropylamine compound according to claim 1, in association with a pharmaceutically acceptable carrier, said compound being presented in an amount effective to provide β-receptor blocking activity in said mammal.

13. A pharmaceutical preparation according to claim 5, wherein the β-receptor blocking compound of claim 1 is present in association with a liquid pharmaceutically acceptable carrier.

14. A compound according to one or more of claims 1 to 10 for use in treatment of acute myocardial infarction, said treatment comprising administering to a mammal suffering from such infarction a therapeutically effective amount of such compound by way of intravenous administration.

15. A compound according to claim 14 for use in treatment by which the compound is administered in an amount of 0.5-1.5 µmoles per kg body weight and minute.

## Claims (Claims for the following Contracting State(s) : AT)

1. Process for the preparation of a new amine compound of the formula I 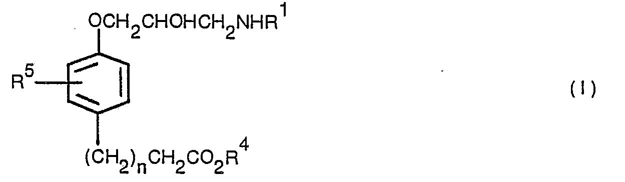 wherein R' is selected from the group consisting of isopropyl and tert. butyl, R⁵ is selected from the group consisting of hydrogen, halogen, lower alkyl having up to 4 carbon atoms, lower alkenyl having up to 4 carbon atoms, and lower alkoxy having up to 4 carbon atoms, R⁴ is selected from the group consisting of alkyl having up to 7 carbon atoms, alkoxyalkyl having up to 8 carbon atoms, cycloalkyl having 3-6 carbon atoms, phenyl, arylalkyl having up to 10 carbon atoms, and hydroxyalkyl having up to 7 carbon atoms, and n is an integer 1 or 2, or a therapeutically acceptable salt thereof, characterized in that
a) a compound of the formula II 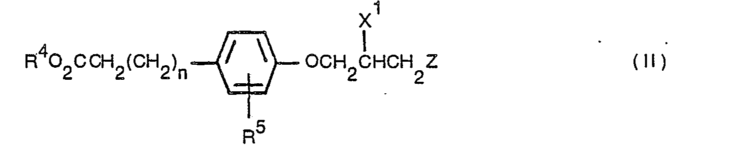 wherein R⁴, R⁵ and n have the same meanings as given above and X¹ is a hydroxy group and Z is a reactive, esterified hydroxy group, or X¹ and Z together form an epoxy group, is reacted with an amine of the formula III 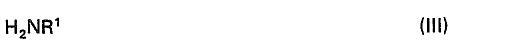 wherein R¹ has the meaning given above:
b) a compound of the formula IV 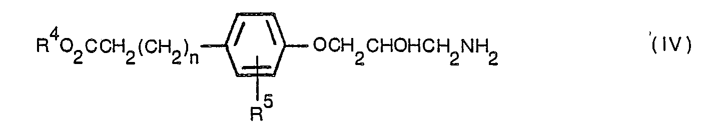 wherein R⁴, R⁵ and n have the same meanings as given above, is reacted with a compound of the formula V 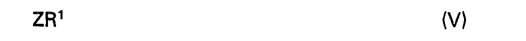 wherein R¹ and Z have the same meanings as given above; or
c) a compound of the formula VI 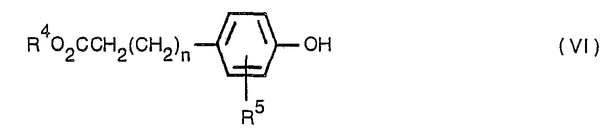 wherein R⁴, R⁵ and n have the same meanings as given above, is reacted with a compound of formula VII 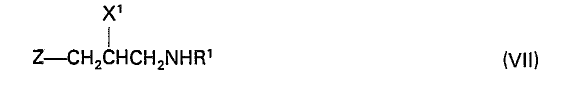 wherein R¹ and X¹ have the same meanings as given above; or
d) a compound of the formula VI 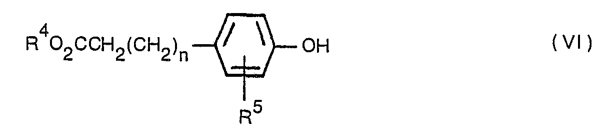 wherein R⁴, R⁵ and n have the same meanings as given above, is reacted with a compound of the formula VIII 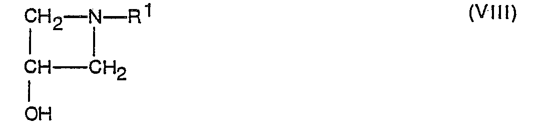 wherein R¹ has the same meaning as given above;
e) from a compound of the formula I, wherein R¹, R⁴, R⁵ and n have the same meanings as above, and which compound has a splitable residue at the nitrogen atom of the amino group and/or has a splitable residue at the hydroxy groups, this residue is split off; or
f) a Schiff's base of the formula X or XI 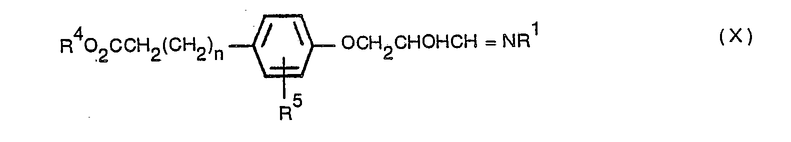 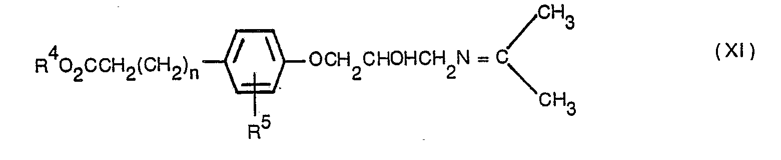 or a cyclic tautomer of formula XII corresponding to the compound of formula XI 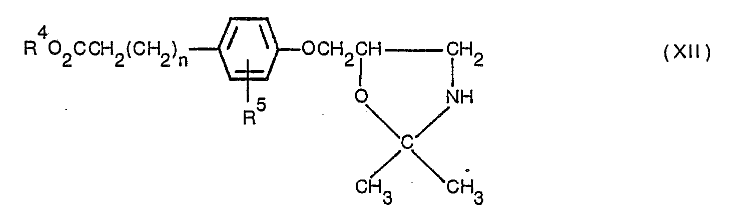 wherein R¹, R⁴, R⁵ and n have the same meanings as above, whereby the compounds XI and XII may be present simultaneously, is reduced, or
g) in a compound of the formula XIII 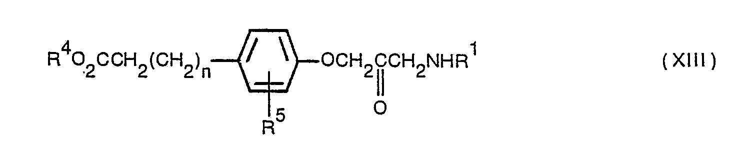 wherein R¹, R⁴, R⁵ and n have the same meanings as above, the oxo group is reduced to a hydroxy group,
h) in a compound of the formula XIV 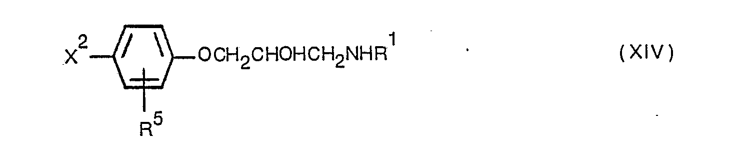 wherein R¹ and R⁵ have the same meanings as given above, X² is a residue transformable into R⁴O₂CCH₂(CH₂)ₙ- wherein R⁴ and n have the above meanings, X² is transformed into R⁴O₂CCH₂(CH₂)ₙ-, or
i) reducing a compound of formula XV 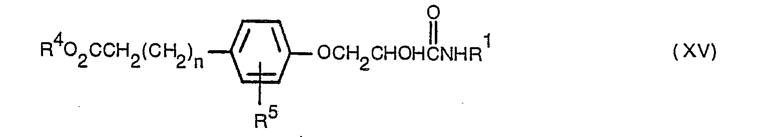 wherein R¹, R⁴, R⁵ and n have the same meanings as above; or.
k) in a compound of the formula XIX 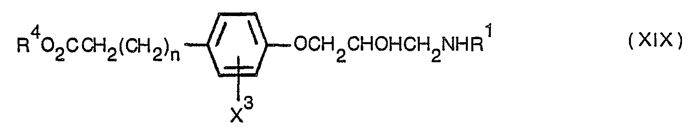 wherein R', R⁴ and n have the same meanings as given above, and X³ is a residue transformable into R⁵, X³ is transformed into R⁵;
I) hydrogenating a compound of the formula 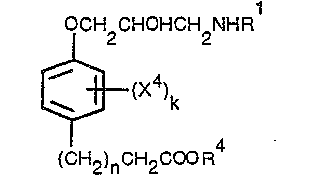 wherein R', R⁴ and n have the meanings given above, and X⁴ denotes a residue that can be split off, and k is an integer 1 to 4, to split X³ off, to the formation of compounds wherein R⁵ is hydrogen; and if desired, substituents are introduced, split off, or reacted in the compounds of the definition of the end product, or compounds obtained are transformed into other end products and/or isomer mixtures obtained are separated into pure isomers, and/or racemates obtained are separated into optical antipodes and/or free bases obtained are transformed into their therapeutically acceptable salt or salts obtained are transformed into their free bases.

2. A process according to claim 1, for preparation of a compound wherein R' is selected from the group consisting of isopropyl and tert. butyl, R⁵ is selected from the group consisting of hydrogen, halogen, alkyl having up to 4 carbon atoms, alkenyl having up to 4 carbon atoms, and alkoxy having up to 4 carbon atoms, R⁴ is selected from the group consisting of alkyl having up to 7 carbon atoms, alkoxyalkyl having up to 8 carbon atoms, cycloalkyl having 3 to 6 carbon atoms, arylalkyl having up to 10 carbon atoms and hydroxyalkyl having up to 7 carbon atoms, and n is an integer 1 or 2, or a therapeutically acceptable salt of such a compound.

3. A process according to claim 2 characterized in preparing methyl 3-[4-(2-hydroxy-3-isopropyl- aminopropoxy)phenyl]propanoate, or a therapeutically acceptable salt thereof.

4. A process according to claim 2 characterized in preparing ethyl 3-[4-(2-hydroxy-3-isopropyl- aminopropoxy)phenyl]propanoate, or a therapeutically acceptable salt thereof.

5. A process according to claim 2 characterized in preparing methyl 3-[4-(2-hydroxy-3-isopropyl- aminopropoxy)-3-methoxyphenyl]-propanoate, or a therapeutically acceptable salt thereof.

6. A process according to claim 2 characterized in preparing 2-methoxyethyl 4-[4-(2-hydroxy-3-isopropylaminopropoxy)phenyl]-butanoate, or a therapeutically acceptable salt thereof.

7. A process according to claim 2 characterized in preparing ethyl 3-[3-chloro-4-(2-hydroxy-3-isopropylaminopropoxy)phenyl]-propanoate, or a therapeutically acceptable salt thereof.

8. A process according to claim 2 characterized in preparing ethyl 3-[3-fluoro-4-(2-hydroxy-3-isopropylaminopropoxy)phenyl]-propanoate, or a therapeutically acceptable salt thereof.

9. A process according to claim 2 characterized in preparing ethyl 3-[4-(2-hydroxy-3-isopropyl- aminopropoxy)-3-methoxyphenyl]-propanoate, or a therapeutically acceptable salt thereof.

10. A process according to claim 2 characterized in preparing ethyl 3-[3-ethoxy-4-(2-hydroxy-3-isopropylaminopropoxy)phenyl]-propanoate, or a therapeutically acceptable salt thereof.

## Patentansprüche (Patentansprüche für folgende(n) Vertragsstaat(en) : BE CH DE FR GB IT LI LU NL SE)

1. Verbindung der Formel I 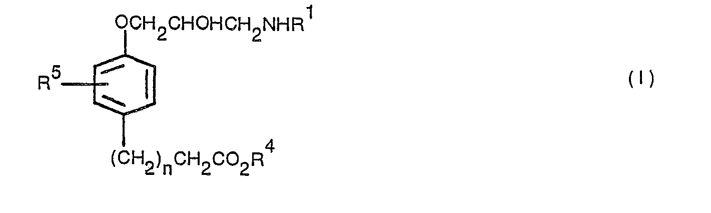 worin R¹ aus der Gruppe lsopropyl und Tertiärbutyl ausgewählt ist, R⁵ aus der Gruppe Wasserstoff, Halogen, niedermolekulares Alkyl mit bis zu 4 Kohlenstoffatomen, niedermolekulares Alkenyl mit bis zu 4 Kohlenstoffatomen und niedermolekulares Alkoxy mit bis zu 4 Kohlenstoffatomen ausgewählt ist, R⁴ aus der Gruppe Alkyl mit bis zu 7 Kohlenstoffatomen, Alkoxyalkyl mit bis zu 8 Kohlenstoffatomen, Cycloalkyl mit 3 bis 6 Kohlenstoffatomen, Phenyl, Arylalkyl mit bis zu 10 Kohlenstoffatomen und Hydroxyalkyl mit bis zu 7 Kohlenstoffatomen ausgewählt ist und n die ganze Zahl 1 oder 2 bedeutet, oder ein therapeutisch verträgliches Salz einer solchen Verbindung.

2. Verbindung nach Anspruch 1, worin R^{I} aus der Gruppe Isopropyl und Tertiärbutyl ausgewählt ist, R⁵ aus der Gruppe Wasserstoff, Halogen, Alkyl mit bis zu 4 Kohlenstoffatomen, Alkenyl mit bis zu 4 Kohlenstoffatomen und Alkoxy mit bis zu 4 Kohlenstoffatomen ausgewählt ist, R⁴ aus der Gruppe Alkyl mit bis zu 7 Kohlenstoffatomen, Alkoxyalkyl mit bis zu 8 Kohlenstoffatomen, Cycloalkyl mit 3 bis 6 Kohlenstoffatomen, Arylalkyl mit bis zu 10 Kohlenstoffatomen und Hydroxyalkyl mit bis zu 7 Kohlenstoffatomen ausgewählt ist und n die ganze Zahl 1 oder 2 bedeutet, oder ein therapeutisch verträgliches Salz einer solchen Verbindung.

3. Methyl-3-[4-(2-hydroxy-3-isopropylaminopropoxy)-phenyl]-propanoat oder ein therapeutisch verträgliches Salz desselben.

4. Äthyl-3-[4-(2-hydroxy-3-isopropylaminopropoxy)-phenyl]-propanoat oder ein therapeutisch verträgliches Salz desselben.

5 Methyl-3-[4-(2-hydroxy-3-isopropylaminopropoxy)-3-methoxyphenyl]-propanoat oder ein therapeutisch verträgliches Salz desselben.

6. 2-Methoxyäthyl-4-[4-(2-hydroxy-3-isopropylaminopropoxy)phenyl]-butanoat oder ein therapeutisch verträgliches Salz desselben.

7. Äthyl-3-[3-chlor-4-(2-hydroxy-3-isopropylaminopropoxy)phenyl]-propanoat oder ein therapeutisch verträgliches Salz desselben.

8. Äthyl-3-[3-fluor-4-(2-hydroxy-3-isopropylaminopropoxy)phenyl]-propanoat oder ein therapeutisch verträgliches Salz desselben.

9. Äthyl-3-[4-(2-hydroxy-3-isopropylaminopropoxy)-3-methoxyphenyl]-propanoat oder ein therapeutisch verträgliches Salz desselben.

10. Äthyl-3-[3-äthoxy-4-(2-hydroxy-3-isopropylaminopropoxy)phenyl]-propanoat oder ein therapeutisch verträgliches Salz desselben.

11. Verfahren zur Herstellung einer neuen Aminverbindung der Formel I 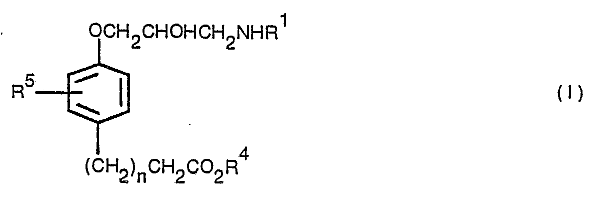 worin R¹ aus der Gruppe Isopropyl und Tertiärbutyl ausgewählt ist, R⁵ aus der Gruppe Wasserstoff, Halogen, niedermolekulares Alkyl mit bis zu 4 Kohlenstoffatomen, niedermolekulares Alkenyl mit bis zu 4 Kohlenstoffatomen und niedermolekulares Alkoxy mit bis zu 4 Kohlenstoffatomen ausgewählt ist, R⁴ aus der Gruppe Alkyl mit bis zu 7 Kohlenstoffatomen, Alkoxyalkyl mit bis zu 8 Kohlenstoffatomen, Cycloalkyl mit 3 bis 6 Kohlenstoffatomen, Phenyl, Arylalkyl mit bis zu 10 Kohlenstoffatomen und Hydroxyalkyl mit bis zu 7 Kohlenstoffatomen ausgewählt ist und n eine ganze Zahl 1 oder 2 bedeutet, oder eines therapeutisch verträglichen Salzes derselben, dadurch gekennzeichnet, daß man
a) eine Verbindung der Formel II 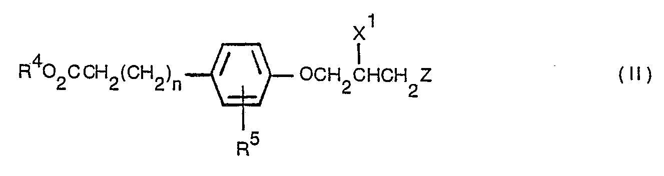 worin R⁴, R⁵ und n die obige Bedeutung haben und X' eine Hydroxylgruppe und Z eine reaktive veresterte Hydroxylgruppe bedeutet oder X' und Z zusammengenommen eine Epoxygruppe bilden, mit einem Amin der Formel III 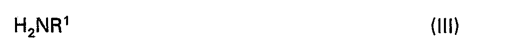 worin R^{I} die obige Bedeutung hat; umsetzt
b) eine Verbindung der Formel IV 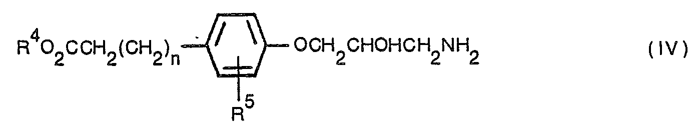 worin R⁴, R⁵ und n die obige-Bedeutung haben, mit einer Verbindung der Formel V 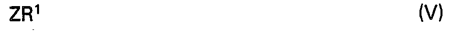 worin R¹ und Z die obige Bedeutung haben, umsetzt; oder
c) eine Verbindung der Formel VI 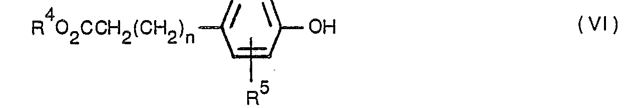 worin R⁴, R⁵ und n die obige Bedeutung haben, mit einer Verbindung der Formel VII 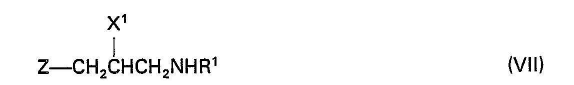 worin R¹ und X¹ die obige Bedeutung haben, umsetzt; oder
d) eine Verbindung der Formel VI 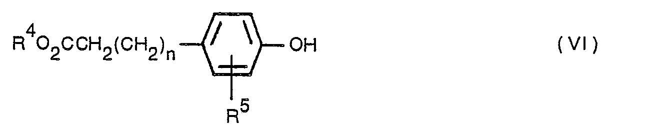 worin R⁴, R⁵ und n die obige Bedeutung haben, mit einer Verbindung der Formel VIII 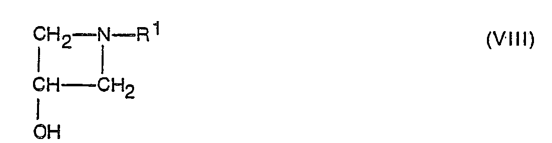 worin R¹ die obige Bedeutung hat, umsetzt;
e) von einer Verbindung der Formel I, worin R¹, R⁴, R⁵ und n die obige Bedeutung haben und welche Verbindung einen abspaltbaren Rest am Stickstoffatom der Aminogruppe und/oder einen abspaltbaren Rest an den Hydroxylgruppen hat, diesen Rest abspaltet; oder
f) eine Schiff'sche Base der Formel X oder XI 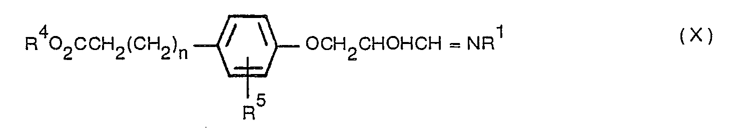 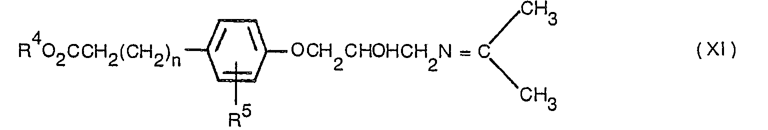 oder ein zyklisches Tautomeres der Formel XII entsprechend der Verbindung der Formel XI 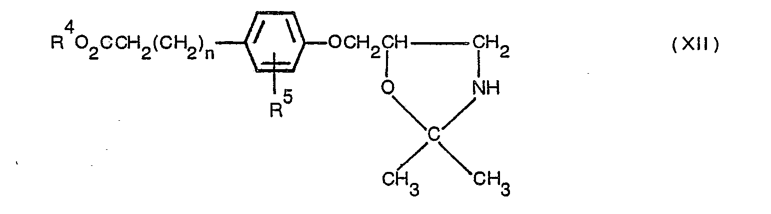 worin R¹, R⁴, R⁵ und n die obige Bedeutung haben, wobei die Verbindungen XI und XII gleichzeitig vorliegen können, reduziert, oder
g) in einer Verbindung der Formel XIII 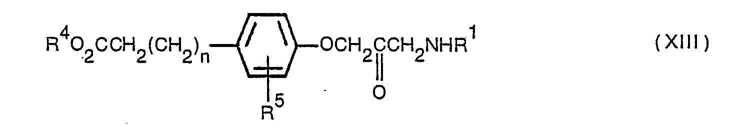 worin R¹, R⁴, R⁵ und n die obige Bedeutung haben, die Oxogruppe zu einer Hydroxylgruppe reduziert,
h) in einer Verbindung der Formel XIV 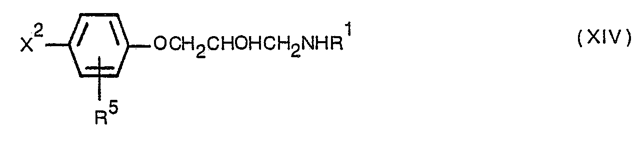 worin R¹ und R⁵ die obige Bedeutung haben, X² ein in R⁴O₂CCH₂(CH₂)ₙ-, worin R⁴ und n die obige Bedeutung haben, überführbarer Rest ist, X² in IR⁴O₂CCH₂(CH₂)ₙ- überführt; oder
i) eine Verbindung der Formel XV 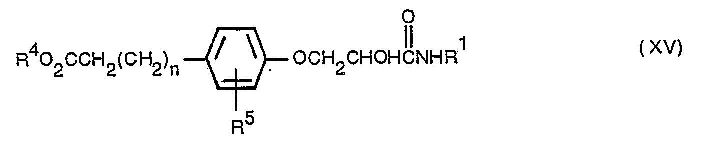 worin R¹, R⁴, R⁵ und n die obige Bedeutung haben, reduziert; oder
k) in einer Verbindung der Formel XIX 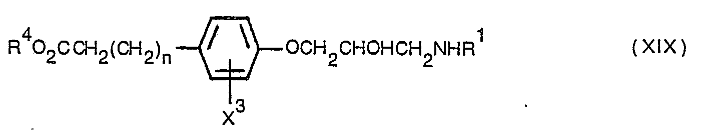 worin R¹, R⁴ und n die obige Bedeutung haben und X³ ein in R⁵ überführbarer Rest ist, X³ in R⁵ überführt;
I) eine Verbindung der Formel 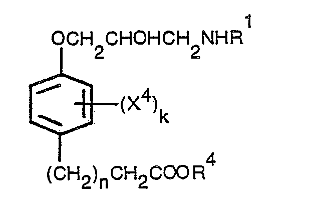 worin R¹, R⁴ und n die obige Bedeutung haben und X⁴ einen Rest bedeutet, der abgespalten werden kann, und k eine ganze Zahl von 1 bis 4 ist, hydriert und so X³ unter Bildung von Verbindungen, worin R⁵ Wasserstoff ist, abspaltet; und gegebenenfalls in den Verbindungen gemäß der Definition des Endproduktes Substituenten einführt, abspaltet oder umsetzt oder erhaltene Verbindungen in andere Endprodukte überführt und/oder erhaltene Isomerengemische in reine Isomere auftrennt und/oder erhaltene Racemate in optische Antipoden auftrennt und/oder erhaltene freie Basen in ihr therapeutisch verträgliches Salz überführt oder erhaltene Salze in ihre freien Basen überführt.

12. Pharmazeutisches Präparat für intravenöse Verabreichung zur Behandlung von akutem Myocardinfarkt bei einem Säugetier, enthaltend wenigstens eine β-rezeptorblockierende Phenoxy-Hydroxypropylaminverbindung nach Anspruch 1 in Verbindung mit einem pharmazeutisch verträglichen Trägermaterial, wobei diese Verbindung in einer wirksamen Menge vorliegt, um bei dem Säugetier eine β-rezeptorblockierende Aktivität zu ergeben.

13. Pharmazeutisches Präparat nach Anspruch 5, worin die β-rezeptorblockierend Verbindung nach Anspruch 1 in Verbindung mit einem flussigen pharmazeutisch verträglichen Trägermaterial vorliegt.

14. Verbindung nach einem oder mehreren der Ansprüche 1 bis 10 für die Verwendung bei der Behandlung von akutem Myocardinfarkt, wobei diese Behandlung eine Verabreichung einer therapeutisch wirksamen Menge einer solchen Verbindung auf intravenösem Weg an ein unter solchem Infarkt leidendes Säugetier einschließt.

15. Verbindung nach Anspruch 14 für die Verwendung bei einer Behandlung, bei der die Verbindung in einer Menge von 0,5 bis 1,5 µMol/Kg Körpergewicht und Minute verabreicht wird.

## Patentansprüche (Patentansprüche für folgende(n) Vertragsstaat(en) : AT)

1. Verfahren zur Herstellung einer neuen Aminverbindung der Formel I 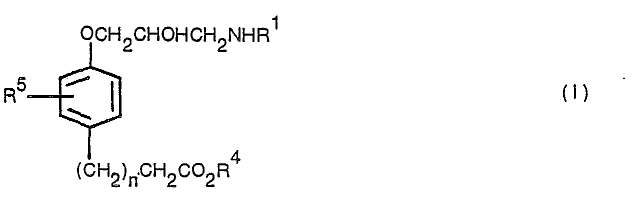 worin R¹ aus der Gruppe Isopropyl und Tertiärbutyl ausgewählt ist, R⁵ aus der Gruppe Wasserstoff, Halogen, niedermolekulares Alkyl mit bis zu 4 Kohlenstoffatomen, niedermolekulares Alkenyl mit bis zu 4 Kohlenstoffatomen und niedermolekulares Alkoxy mit bis zu 4 Kohlenstoffatomen ausgewählt ist, R⁴ aus der Gruppe Alkyl mit bis zu 7 Kohlenstoffatomen, Alkoxyalkyl mit bis zu 8 Kohlenstoffatomen, Cycloalkyl mit 3 bis 6 Kohlenstoffatomen, Phenyl, Arylalkyl mit bis zu 10 Kohlenstoffatomen und Hydroxyalkyl mit bis zu 7 Kohlenstoffatomen ausgewählt ist und n die ganze Zahl 1 oder 2 bedeutet, oder eines therapeutisch verträgliches Salzes derselben, dadurch gekennzeichnet, daß man
a) eine Verbindung der Formel II 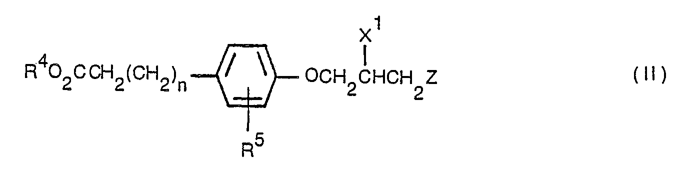 worin R⁴, R⁵ und n die obige Bedeutung haben und X¹ eine Hydroxylgruppe und Z eine reaktive veresterte Hydroxylgruppe bedeutet oder X¹ und Z zusammengenommen ein Epoxygruppe bilden, mit einem Amin der Formel III 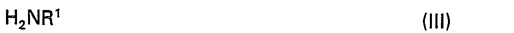 worin R¹ die obige Bedeutung hat; umsetzt
b) eine Verbindung der Formel IV 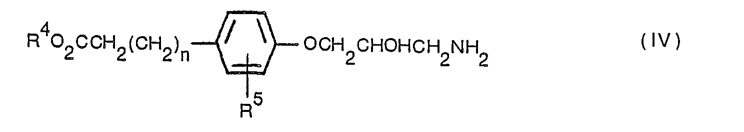 worin R⁴, R⁵ und n die obige Bedeutung haben, mit einer Verbindung der Formel V 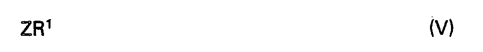 worin R¹ und Z die obige Bedeutung haben, umsetzt; oder
c) eine Verbindung der Formel VI 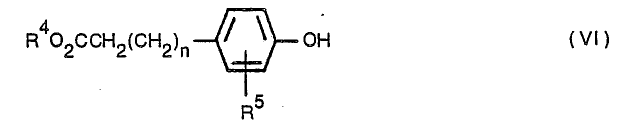 worin R⁴, R⁵ und n die obige Bedeutung haben, mit einer Verbindung der Formel VII 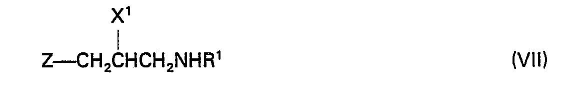 worin R¹ und X¹ die obige Bedeutung haben, umsetzt; oder
d) eine Verbindung der Formel VI 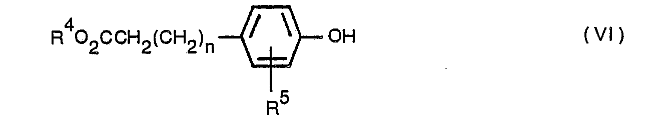 worin R⁴, R⁵ und n die obige Bedeutung haben, mit einer Verbindung der Formel VIII 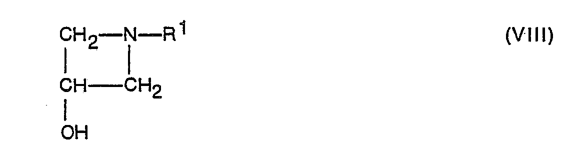 worin R¹ die obige Bedeutung hat, umsetzt;
e) von einer Verbindung der Formel I, worin R¹, R⁴, R⁵ und n die obige Bedeutung haben und welche Verbindung einen abspaltbaren Rest am Stickstoffatom der Aminogruppe und/oder einer abspaltbaren-Rest an den Hydroxylgruppen hat, diesen Rest abspaltet; oder
f) eine Schiff'sche Base der Formel X oder XI 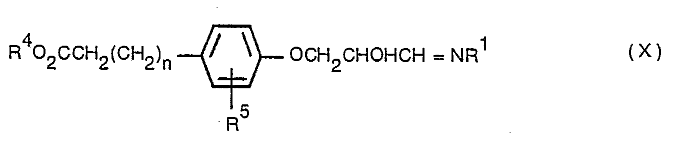 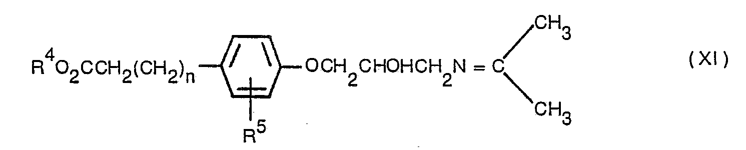 oder ein zyklisches Tautomeres der Formel XII entsprechend der Verbindung der Formel XI 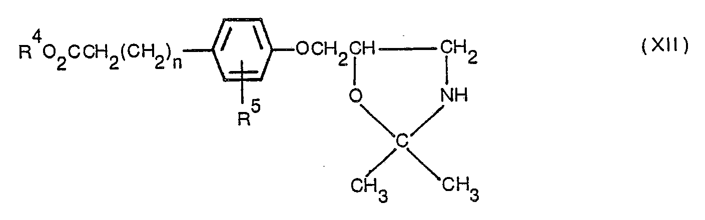 worin R¹, R⁴, R⁵ und n die obige Bedeutung haben, wobei die Verbindungen XI und XII gleichzeitig vorliegen können, reduziert, oder
g) in eine Verbindung der Formel XIII 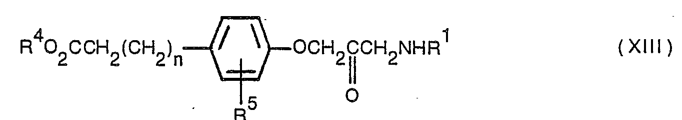 worin R¹, R⁴, R⁵ und n die obige Bedeutung haben, die Oxogruppe zu einer Hydroxylgruppe reduziert,
h) in einer Verbindung der Formel XIV 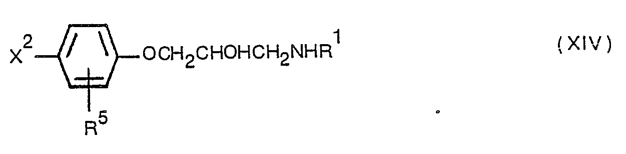 worin R¹ und R⁵ die obige Bedeutung haben, X² ein in R⁴O₂CCH₂(CH₂)ₙ-, worin R⁴ und n die obige Bedeutung haben, überführbarer Rest ist, X² in R⁴O₂CCH₂(CH₂)ₙ- überführt; oder
i) eine Verbindung der Formel XV 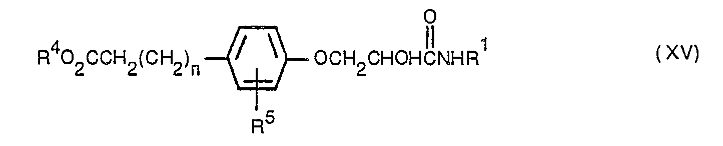 worin R¹, R⁴, R⁵ und n die obige Bedeutung haben, reduziert; oder
k) in einer Verbindung der Formel XIX 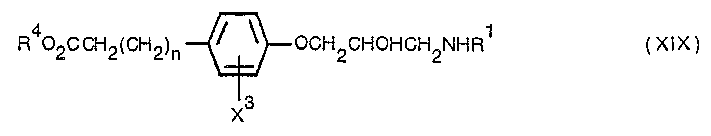 worin R¹, R⁴ und n die obige Bedeutung haben und X³ ein in R⁵ überführbarer Rest ist, X³ in R⁵ überführt;
I) eine Verbindung der Formel 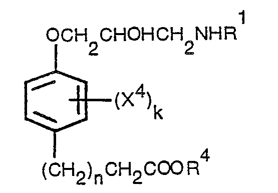 worin R¹, R⁴ und n die obige Bedeutung haben und X⁴ einen Rest bedeutet, der abgespalten werden kann, und k eine ganze Zahl von 1 bis 4 ist, hydriert und so X³ unter Bildung von Verbindung, worin R⁵ Wasserstoff ist, abspaltet; und gegebenenfalls in den Verbindungen gemäß der Definition des Endproduktes Substituenten einführt, abspaltet oder umsetzt oder erhaltene Verbindungen in andere Endprodukte überführt und/oder erhaltene Isomerengemische in reine Isomere auftrennt und/oder erhaltene Racemate in optische Antipoden auftrennt und/oder erhaltene freie Basen in ihr therapeutisch verträgliches Salz überführt oder erhaltene Salze in ihre freien Basen überführt.

2. Verfahren nach Anspruch 1 zur Herstellung einer Verbindung, in der R¹ aus der Gruppe Isopropyl und Tertiarbutyl ausgewählt ist, R⁵ aus der Gruppe Wasserstoff, Halogen, Alkyl mit bis zu 4 Kohlenstoffatomen, Alkenyl mit bis zu 4 Kohlenstoffatomen und Alkoxy mit bis zu 4 Kohlenstoffatomen ausgewählt ist, R⁴ aus der Gruppe Alkyl mit bis zu 7 Kohlenstoffatomen, Alkoxyalkyl mit bis zu 8 Kohlenstoffatomen, Cycloalkyl mit 3 bis 6 Kohlenstoffatomen, Arylalkyl mit bis zu 7 Kohlenstoffatomen und Hydroxyalkyl mit bis zu 7 Kohlenstoffatomen ausgewählt ist und n die ganze Zahl 1 oder 2 bedeutet, oder eines therapeutisch verträglichen Salzes einer solchen Verbindung.

3. Verfahren nach Anspruch 2, gekennzeichnet durch die Herstellung von Methyl-3-[4-(2-hydroxy-3-isopropylaminopropoxy)-phenyl]-propanoat oder eines therapeutisch verträgliches Salzes hiervon.

4. Verfahren nach Anspruch 2, gekennzeichnet durch die Herstellung von Äthyl-3-[4-(2-hydroxy-3-isopropylaminopropoxy)-phenyl]-propanoat oder eines therapeutisch verträgliches Salzes hiervon.

5. Verfahren nach Anspruch 2, gekennzeichnet durch die Herstellung von Methyl-3-[4-(2-hydroxy-3-isopropylaminopropoxy)-3-methoxyphenyl]-propanoat oder eines therapeutisch verträgliches Salzes hiervon.

6. Verfahren nach Anspruch 2, gekennzeichnet durch die Herstellung von 2-Methoxyäthyl-4-[4-(2-hydroxy-3-isopropylaminopropoxy)phenyli-butanoat oder eines therapeutisch verträglichen Salzes hiervon.

7. Verfahren nach Anspruch 2, gekennzeichnet durch die Herstellung von Äthyl-3-[3-chlor-4-(2-hydroxy-3-isopropylaminopropoxy)phenyl]-propanoat oder eines therapeutisch verträgliches Salzes hiervon.

8. Verfahren nach Anspruch 2, gekennzeichnet durch die Herstellung von Äthyl-3-[3-flour-4-(2-hydroxy-3-isopropylaminopropoxy)phenyl]-propanoat oder eines therapeutisch verträgliches Salzes hiervon.

9. Verfahren nach Anspruch 2, gekennzeichnet durch die Herstellung von Äthyl-3-[4-(2-hydroxy-3-isopropylaminopropoxy)-3-methoxyphenyl]-propanoat oder eines therapeutisch verträgliches Salzes hiervon. -

10. Verfahren nach Anspruch 2, gekennzeichnet durch die Herstellung von Äthyl-3-[3-äthoxy-4-(2-hydroxy-3-isopropylaminopropoxy)phenyl]-propanoat oder eines therapeutisch verträgliches Salzes hiervon.

## Revendications (Revendications pour l'(les) Etat(s) contractant(s) suivant(s) : BE CH DE FR GB IT LI LU NL SE)

1. Composé de formule I 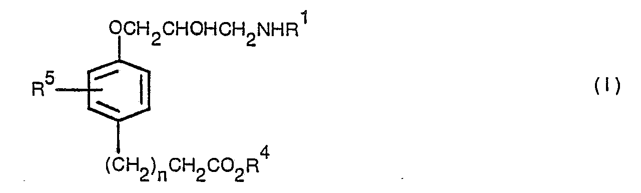 dans laquelle R^{I} est choisi dans le groupe constitué par l'isopropyle et le tertiobutyle, R⁵ est choisi dans le groupe constitué par l'hydrogène, un halogène, un alkyle inférieur ayant jusqu'à 4 atomes de carbone, un alcényle inférieur ayant jusqu'à 4 atomes de carbone et un alcoxy inférieur ayant jusqu'à 4 atomes de carbone, R⁴ est choisi dans le groupe constitué par un alkyle ayant jusqu'à 7 atomes de carbone, un alcoxyalkyle ayant jusqu'à 8 atomes de carbone, un cycloalkyle ayant 3-6 atomes de carbone, un phényle, un arylalkyle ayant jusqu'à 10 atomes de carbone et un hydroxyalkyle ayant jusqu'à 7 atomes de carbone, et n est un nombre entier égal à 1 ou 2, ou un sel thérapeutiquement acceptable d'un tel composé.

2. Composé selon la revendication 1, dans lequel R^{I} est choisi dans le groupe constitué par l'isoproyle et le tertiobutyle, R⁵ est choisi dans le groupe constitué par l'hydrogène, un halogène, un alkyle ayant jusqu'à 4 atomes de carbone, un alcényle ayant jusqu'à 4 atomes de carbone et un alcoxy ayant jusqu'à 4 atomes de carbone, R⁴ est choisi dans le groupe constitué par un alkyle ayant jusqu'à 7 atomes de carbone, un alcoxyalkyle ayant jusqu'à 8 atomes de carbone, un cycloalkyle ayant 3-6 atomes de carbone, un arylalkyle ayant jusqu'à 10 atomes de carbone et un hydroxyalkyle ayant jusqu'à 7 atomes de carbone, et n est un nombre entier égal à 1 ou 2, ou un sel thérapeutiquement acceptable d'un tel composé.

3. 3-[4-(2-Hydroxy-3-isopropylaminopropoxy)phényl]-propanoate de méthyle, ou un des ses sels thérapeutiquement acceptables.

4. 3-[4-(2-Hydroxy-3-isopropylaminopropoxy)phényll-propanoate d'éthyl, ou un de ses sels thérapeutiquement acceptables.

5. 3-[4-(2-Hydroxy-3-ispropylaminopropoxy)-3-méthoxyphényl]-propanoate de méthyle, ou un de ses sels thérapeutiquement acceptables.

6. 4-[4-(2-Hydroxy-3-isopropylaminopropoxy)phényl]-butanoate de 2-méthoxyéthyle, ou un de ses sels thérapeutiquement acceptables.

7. 3-[3-Chloro-4-(2-hydroxy-3-isopropylaminopropoxy)phényl]-propanoate d'éthyle, ou un de ses sels thérapeutiquement acceptables.

8. 3-[3-Fluoro-4-(2-hydroxy-3-isopropylaminopropoxy)phényl]-propanoate d'éthyle, ou un de ses sels thérapeutiquement acceptables.

9. 3-[4-(2-Hydroxy-3-isopropylaminopropoxy)-3-méthoxyphényl]-propanoate d'éthyle, ou un de ses sels thérapeutiquement acceptables.

10. 3-[3-Ethoxy-4-(2-hydroxy-3-isopropylaminopropoxy)phényl]-propanoate d'éthyle, ou un de ses sels thérapeutiquement acceptables.

11. Procédé pour la préparation d'une nouvelle amine de formule I 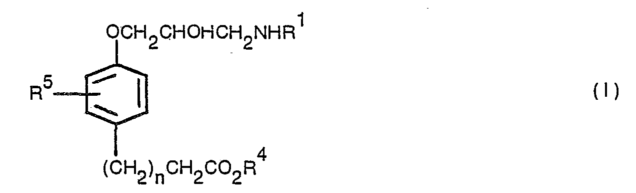 dans laquelle R^{I} est choisi dans le groupe constitué par l'isopropyle et le tertiobutyle, R⁵ est choisi dans le groupe constitué par l'hydrogène, un halogène, un alkyle inférieur ayant jusqu'à 4 atomes de carbone, un alcényle inférieur ayant jusqu'à 4 atomes de carbone et un alcoxy inférieur ayant jusqu'à 4 atomes de carbone, R⁴ est choisi dans le groupe constitué par un alkyle ayant jusqu'à 7 atomes de carbone, un alcoxyalkyle ayant jusqu'à 8 atomes de carbone, un cycloalkyle ayant 3-6 atomes de carbone, un phényle, un arylalkyle ayant jusqu'à 10 atomes de carbone et un hydroxyalkyle ayant jusqu'à 7 atomes de carbone, et n est un nombre entier égal à 1 ou 2, ou l'un de ses sels thérapeutiquement acceptables, caractérisé en ce que
a) on fait réagir un composé de formule Il 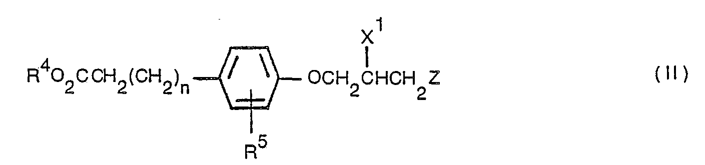 dans laquelle R⁴, R⁵ et n ont les mêmes significations que celles données ci-dessus et X^{I} est un groupe hydroxy et Z est un groupe hydroxy estérifié réactif, ou X¹ et Z forment ensemble un groupe époxy, avec une amine de formule III 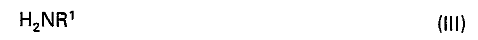 dans laquelle R¹ a la signification donnée ci-dessus;
b) on fait réagir un composé de formule IV 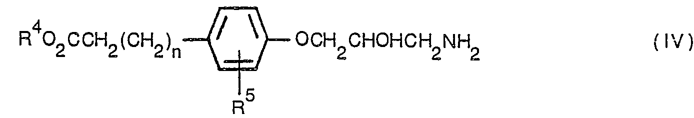 dans laquelle R⁴, R⁵ et n one les mêmes significations que celles données ci-dessus avec un composé de formule V 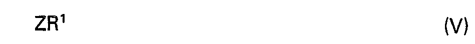 dans laquelle R¹ et Z ont les mêmes significations que celles données ci-dessus; ou
c) on fait réagir un composé de formule VI 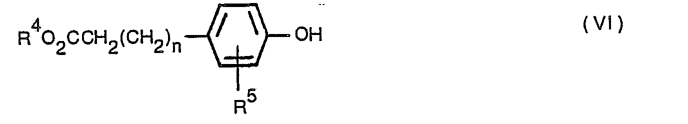 dans laquelle R⁴, R⁵ et n ont les mêmes significations que celles données ci-dessus, avec un composé de formule VII 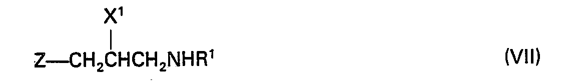 dans laquelle R¹ et X¹ ont les mêmes significations que celles données ci-dessus; ou
d) on fait réagir un composé de formule VI 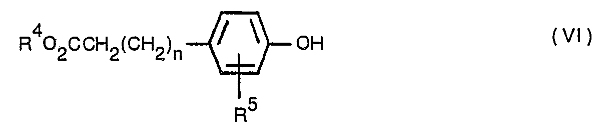 dans laquelle R⁴, R⁵ et n ont les mëmes significations que celles données ci-dessus, avec un composé de formule VIII 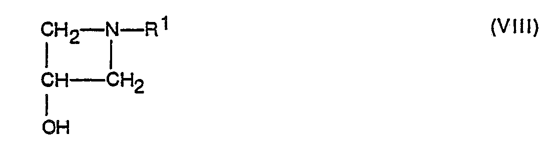 dans laquelle R¹ a la même signification que celle donnée ci-dessus;
e) sur un composé de formule I. dans laquelle R¹, R⁴, R⁵ et n ont la même signification que ci-dessus, et qui a un résidu éliminable sur l'atome d'azote du groupe amino et/ou a un résidu éliminable sur les groupes hydroxy, on effectue l'élimination de ce résidu; ou
f) on réduit une base de Schiff de formule X ou XI 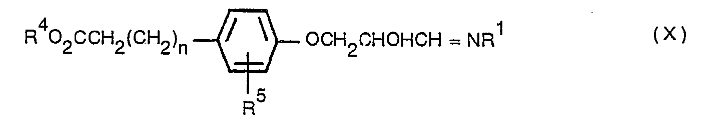 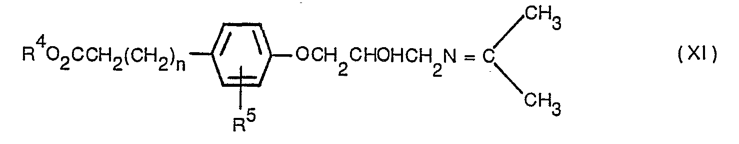 ou un tautomère cyclique de formule XII correspondant au composé de formule XI 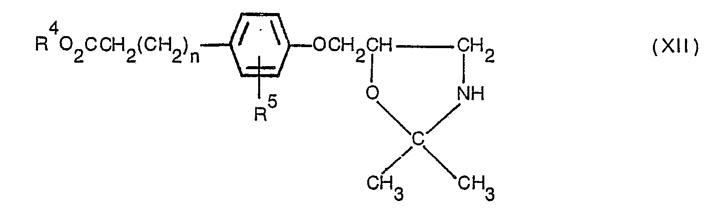 dans lesquelles R^{I}, R⁴, R⁵ et n one les mêmes significations que ci-dessus, les composés XI et XII pouvant être présents simultanément; ou
g) on réduit le groupe carbonyle en hydroxyle dans un composé de formule XIII 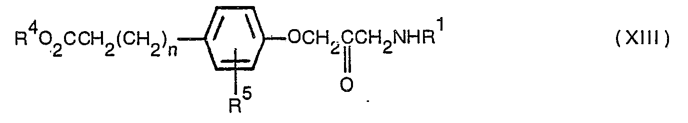 dans laquelle R^{I}, R⁴, R⁵ et n ont les mêmes significations que ci-dessus,
h) on transforme X² en R⁴O₂CCH₂(CH₂)ₙ dans un composé de formule XIV 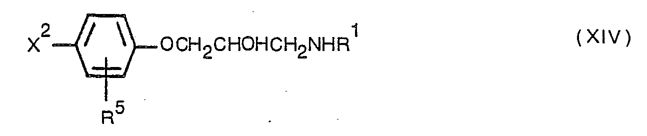 dans laquelle R¹ et R⁵ ont les mêmes significations que celles données ci-dessus, X² est un résidu transformable en R⁴O₂CCH₂(CH₂)ₙ- dans lequel R⁴ et n ont les significations précédentes, ou
i) on réduit un composé de formule XV 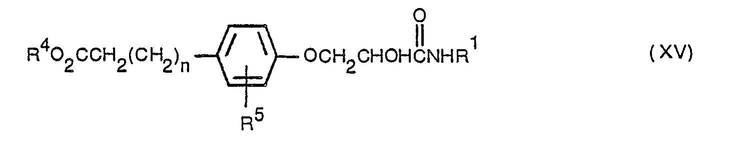 dans laquelle R^{I}, R⁴, R⁵ et n ont les mêmes significatons que ci-dessus; ou
k) on transforme X³ en R⁵ dans un composé de formule XIX 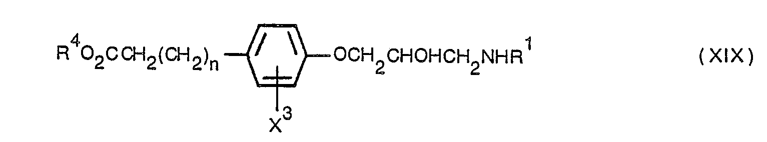 dans laquelle R¹, R⁴ et n ont les mêmes significations que celles données ci-dessus et X³ est un résidu transformable en R⁵,
I) on effectue l'hydrogénation d'un composé de formule 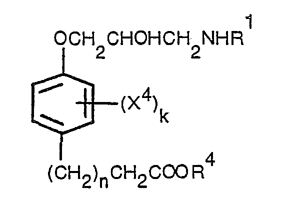 dans laquelle R¹, R⁴ et n ont les mêmes significations que celles données ci-dessus et X⁴ désigne un résidu pouvant être éliminé et k est un nombre entier de 1 à 4, pour effectuer l'élimination de X⁴ et former des composés dans lesquels R⁵ est l'hydrogène; et éventuellement, on introduit, on élimine, ou on fait réagir des substituants pour donner les composés dont la définition est celle du produit final, ou on transforme les composés obtenus en d'autres produits finals et/ou on sépare les mélanges d'isomères obtenus en isomères purs et/ou on sépare les racémates obtenus en antipodes optiques et/ou on transforme les bases libres obtenues en leur sel thérapeutiquement acceptables ou on transforme les sels obtenus en leurs bases libres.

12. Préparation pharmaceutique destinée à l'administration intravieneuse pour le traitement de l'infarctus aigu du myocarde chez le mammifère, comprenant au moins une phénoxyhydroxypropyl- amine β-bloquante selon la revendication 1 associé à un véhicule pharmaceutiquement acceptable, ledit composé étant en quantité suffisante pour avoir une activité β-bloquante chex ledit mammifère.

13. Préparation pharmaceutique selon la revendication 5 contenant le β-bloquant de la revendication 1, associé à un excipient liquide pharmaceutiquement acceptable.

14. Composé selon une ou plusieurs des revendications 1 à 10, utilisable pour le traitement de l'infarctus aigu du myocarde, ledit traitement comprenant l'administration intraveineuse à un mammifère souffrant d'un tel infarctus d'une quantité thérapeutiquement efficace d'un tel composé.

15. Composé selon la revendication 14, utilisable pour un traitement dans lequel le composé est administré en quantité de 0,5-1,5 µmoies par kg de poids corporel et par minute.

## Revendications (Revendications pour l'(les) Etat(s) contractant(s) suivant(s) : AT)

1. Procédé pour la préparation d'une nouvelle amine de formule 1 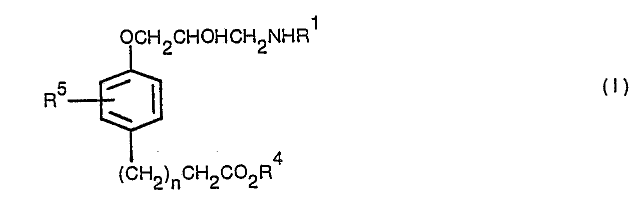 dans laquelle R^{I} est choisi dans le groupe constitué par l'isopropyle et le tertiobutyle, R^{S} est choisi dans le groupe constitué par l'hydrogène, un halogène, un alkyle inférieur ayant jusqu'à 4 atomes de carbone, un alcényle inférieur ayant jusqu'à 4 atomes de carbone et un alcoxy inférieur ayant jusqu'à 4 atomes de carbone, R⁴ est choisi dans le groupe constitué par un alkyle ayant jusqu'à 7 atomes de carbone, un alcoxyalkyle ayant jusqu'à 8 atomes de carbone, un cycloalkyle ayant 3-6 atomes de carbone, un phényle, un arylalkyle ayant jusqu'à 10 atomes de carbone et un hydroxyalkyle ayant jusqu'à 7 atomes de carbone, et n est un nombre entier égal à 1 ou 2, d'un de ses sels thérapeutiquement acceptables, caractérisé en ce que
a) on fait réagir un composé de formule Il 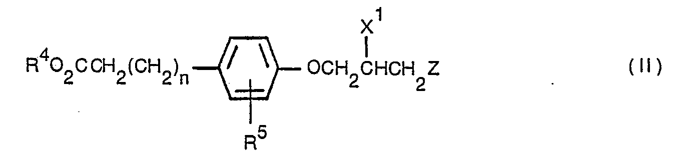 dans laquelle R⁴, R⁵ et n ont les mêmes significations que celles données ci-dessus et X¹ est un groupe hydroxy et Z est un groupe hydroxy estérifié réactif, ou X¹ et Z forment ensemble un groupe époxy, avec une amine de formule III 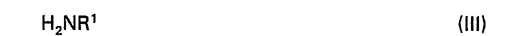 dans laquelle R¹ a la signification donnée ci-dessus;
b) on fait réagir un composé de formule IV 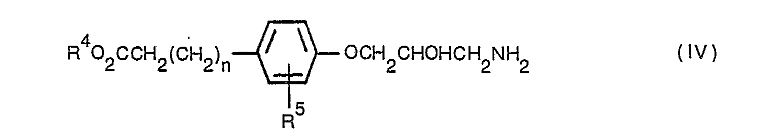 dans laquelle R⁴, R⁵ et n one les mêmes significations que ci-dessus, avec un composé de formule V 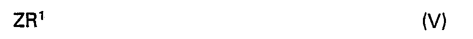 dans laquelle R¹ et Z ont les mêmes significations que celles données ci-dessus; ou
c) on fait réagir un composé de formule VI 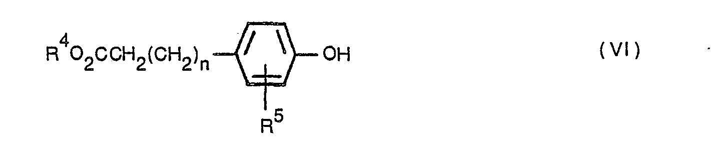 dans laquelle R⁴, R⁵ et n ont les mêmes significations que celles données ci-dessus, avec un composé de formule VII 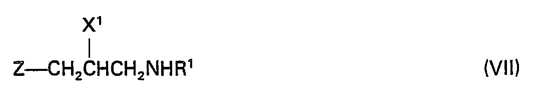 dans laquelle R¹ et X¹ ont les mêmes significations que celles données di-dessus; ou
d) on fait réagir un composé de formule VI 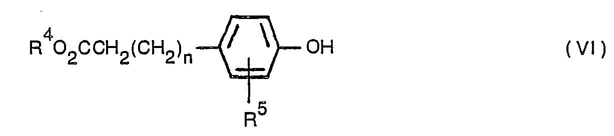 dans laquelle R⁴, R⁵ et n ont les mëmes significations que celles données ci-dessus, avec un composé de formule VIII 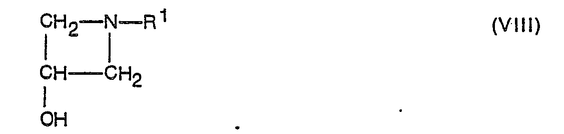 dans laquelle R¹ a la même signification que celle donnée ci-dessus;
e) sur un composé de formule I, dans laquelle R¹, R⁴, R⁵ et n ont la mêmes significations que ci-dessus, et ce composé ayant un résidu éliminable sur l'atome d'azote du groupe amino et/ou a un résidu éliminable sur les groupes hydroxy, on effectue l'élimination de ce résidu; ou
f) on réduit une base de Schiff de formule X ou XI 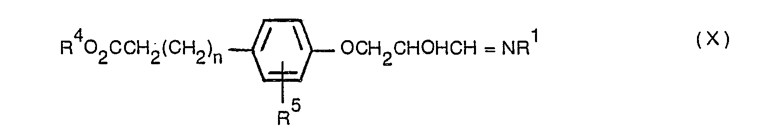 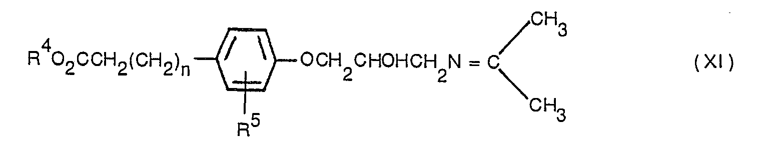 ou un tautomère cyclique de formule XII correspondant au composé de formule XI 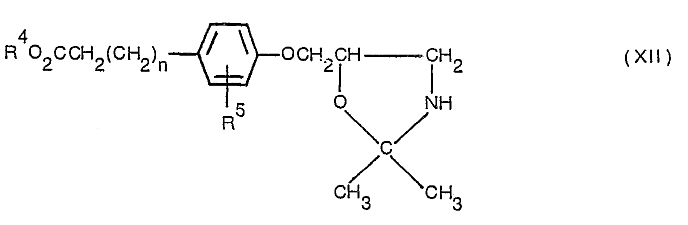 dans lesquelles R^{I}, R⁴, R⁵ et n one les mêmes significations que ci-dessus, les composés XI et XII pouvant être présents simultanément; ou
g) on réduit le groupe carbonyle en hydroxyle dans un composé de formule XIII 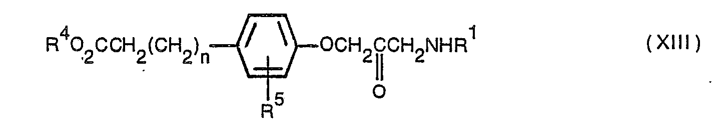 dans laquelle R¹, R⁴, R⁵ et n ont les mêmes significations que ci-dessus,
h) on transforme X² en R⁴O₂CCH₂(CH₂)ₙ- dans un composé de formule XIV 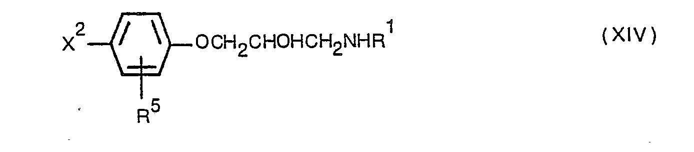 dans laquelle R¹ et R⁵ ont les mêmes significations que celles données ci-dessus et X² est un résidu transformable en R⁴O₂CCH₂(CH₂)ₙ- dans lequel R⁴ et n ont les significations précédentes,
i) on réduit un composé de formule XV 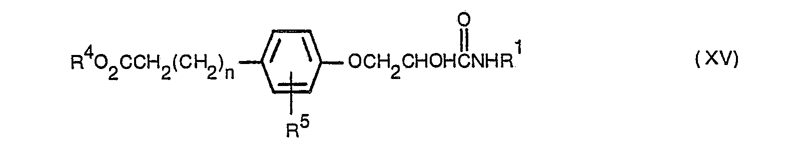 dans laquelle R¹, R⁴, R⁵ et n ont les mêmes significatons que ci-dessus; ou
k) on transforme X³ en R⁵ dans un composé de formule XIX 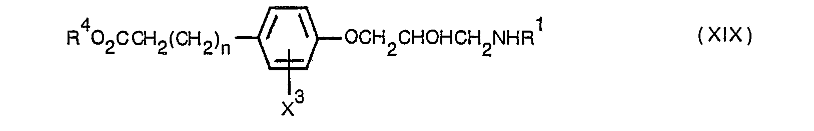 dans laquelle R¹, R⁴ et n ont les mêmes significations que celles données ci-dessus et X³ est un résidu transformable en R⁵,
I) on effectue l'hydrogénation d'un composé de formule 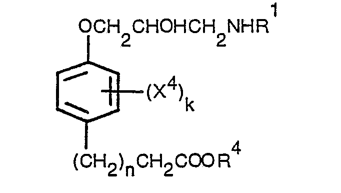 dans laquelle R¹, R⁴ et n ont les mêmes significations que celles données ci-dessus et X⁴ désigne un résidu pouvant être éliminé et k est un nombre entier de 1 à 4, pour effectuer l'élimination de X⁴ et former des composés dans lesquels R⁵ est l'hydrogène; et éventuellement, on introduit, on élimine, ou on fait réagir des substituants pour donner les composés dont la définition est celle du produit final, ou on transforme les composés obtenus en d'autres produits finals et/ou on sépare les mélanges d'isomères obtenus en isomères purs et/ou on sépare les racémates obtenus en antipodes optiques et/ou on transforme les bases libres obtenues en leurs sels theapeutiquement acceptables ou on transforme les sels obtenus en leurs bases libres.

2. Procédé selon la revendication 1, pour la préparation d'un composé dans lequel R¹ est choisi dans le groupe constitué par l'isopropyle et le tertiobutyle, R⁵ est choisi dans le groupe constituté par l'hydrogène, un halogène, un alkyle ayant jusqu'à 4 atomes de carbone, un alcényle ayant jusqu'à 4 atomes de carbone et un alcoxy ayant jusqu'à 4 atomes de carbone, R⁴ est choisi dans le groupe constitué par un alkyle ayant jusqu'à 7 atomes de carbone, un alcoxyalkyle ayant jusqu'à 8 atomes de carbone, un cycloalkyle ayant 3-6 atomes de carbone, un arylalkyle ayant jusqu'à 10 atomes de carbone et un hydroxyalkyle ayant jusqu'à 7 atomes de carbone, et n est un nombre entier égal à 1 ou 2, ou d'un sel thérapeutiquement acceptable de ce composé.

3. Procédé selon la revendication 2, caractérisé en ce que l'on prépare le 3-[4-(2-hydroxy-3-isopropylaminopropoxy)phényl]-propanoate de méthyle, ou un de ses sels thérapeutiquement acceptables.

4. Procédé selon la revendication 2, caractérisé en ce que l'on prépare le 3-[4-(2-hydroxy-3-isopropylaminopropoxy)phényl]-propanoate d'éthyle, ou un de ses sels thérapeutiquement acceptables.

5. Procédé selon la revendication 2, caractérisé en ce que l'on prépare le 3-[4-(2-hydroxy-3- ispropylaminopropoxy)-3-méthoxyphényl]-propanoate de méthyle, ou un de ses sels thérapeutiquement acceptables.

6. Procédé selon la revendication 2, caractérisé en ce que l'on prépare le 4-[4-(2-Hydroxy-3-isopropylaminopropoxy)phényl]-butanoate de 2-méthoxyéthyle, ou un de ses sels thérapeutiquement acceptables.

7. Procédé selon la revendication 2, caractérisé en ce que l'on prépare le 3-[3-chloro-4-(2-hydroxy-3-isopropyiaminopropoxy)phény)]-propanoate d'éthyle, ou un de ses sels thérapeutiquement acceptables.

8. Procédé selon la revendication 2, caractérisé en ce que l'on prépare le 3-[3-fluor-4-(2-hydroxy-3-isopropylaminopropoxy)phényl]-propanoate d'éthyle, ou un de ses sels thérapeutiquement acceptables.

9. Procédé selon la revendication 2, caractérisé en ce que l'on prépare le 3-[4-(2-hydroxy-3-isopropylaminopropoxy)-3-méthoxyphényl]-propanoate d'éthyle, ou un de ses sels thérapeutiquement acceptables.

10. Procédé selon la revendication 2, caractérisé en ce que l'on prépare le 3-[3-éthoxy-4-(2-hydroxy-3-isopropylaminopropoxy)phényl]-propanoate d'éthyle, ou un de ses sels thérapeutiquement acceptables.
